# EUROPÄISCHE PATENTANMELDUNG

(11) **EP 4 759 450 A1**
(43) Veröffentlichungstag der Anmeldung: **17.06.2026**
(21) Anmeldenummer: 24219827.3
(22) Anmeldetag: 13.12.2024
(51) Int. Cl.: B08B 15/02, A61L 2/20, B25J 21/00, F04D 29/058

(54) **VERARBEITUNGSANLAGE MIT EINER KONTROLLIERTEN UMGEBUNG UND VERFAHREN ZUM BEHANDELN DER UMGEBUNG**

(71) Anmelder: SKAN AG, 4123 Allschwil (CH)
(72) Erfinder: Lehmann, Frank, 4102 Binningen (CH); Keller, Adrian, 79588 Efringen-Kirchen (DE); Novák, Martin, 4102 Binningen (CH); Sommerhalder, Matthias Philipp, 4112 Bättwil (CH); Krebsbach, Timo, 4310 Rheinfelden (CH); Bauer, Thomas, 4125 Riehen (CH)
(74) Vertreter: Mertzlufft-Paufler, Cornelius

(57) **Zusammenfassung**

Die Erfindung betrifft eine Verarbeitungsanlage (1) mit einer kontrollierten Umgebung (2) und einer die kontrollierte Umgebung (2) umgebenden Einhausung (3). Der Erfindung liegt die Aufgabe zugrunde, eine Verarbeitungsanlage bereitzustellen, mit welcher die kontrollierte Umgebung in der Verarbeitungsanlage besonders einfach und/oder flexibel behandelt werden kann.

Diese Aufgabe wird insbesondere dadurch gelöst, dass die Verarbeitungsanlage (1) mindestens eine mit der kontrollierten Umgebung (2) fluidal verbundene Ventilationsmaschine (4) mit einem Stator (5) und einem magnetisch berührungslos gelagerten Rotor (6) aufweist.

## Beschreibung

Die Erfindung betrifft eine Verarbeitungsanlage mit einer kontrollierten Umgebung und einer die kontrollierte Umgebung umgebenden Einhausung.

Die Erfindung betrifft ferner ein Verfahren zum Behandeln einer kontrollierten Umgebung einer hier beschriebenen Verarbeitungsanlage.

Verarbeitungsanlagen der hier beschriebenen Art und Verfahren zum Behandeln einer kontrollierten Umgebung in derartigen Verarbeitungsanlagen sind allgemein aus dem Stand der Technik bekannt.

Verarbeitungsanlagen der hier beschriebenen Art können in der Praxis beispielsweise strukturelle und/oder funktionelle Gemeinsamkeiten mit Isolatoren, insbesondere mit ein Dekontaminationssystem aufweisenden Isolatoren, aufweisen. Einfache Isolatoren sind in der Praxis beispielsweise in Form von Handschuhkästen bekannt. In Isolatoren ist es möglich, eine Substanz, ein Material, eine Probe oder ähnliches in einer kontrollierten Umgebung insbesondere stationär zu behandeln. Derartige Isolatoren finden beispielsweise Anwendung im Pharmabereich.

In der hier beschriebenen Verarbeitungsanlage, die strukturelle und/oder funktionelle Gemeinsamkeiten mit den beschriebenen Isolatoren aufweisen kann, ist es ebenfalls möglich, eine Substanz, ein Material, eine Probe oder ähnliches in der kontrollierten Umgebung zu behandeln und insbesondere zu verarbeiten. Optional kann die behandelte Substanz, das behandelte Material, die behandelte Probe oder ähnliches anschließend an einen weiteren Verarbeitungsschritt, der innerhalb oder außerhalb der Verarbeitungsanlage durchgeführt wird, übergeben werden. Die hier beschriebene Verarbeitungsanlage kann analog zu den beschriebenen Isolatoren beispielsweise eine Verarbeitungsanlage sein, die in der pharmazeutischen Verarbeitung Anwendung findet. Zu diesem Zweck kann die kontrollierte Umgebung der Verarbeitungsanlage beispielsweise eine insbesondere kontrollierte Atmosphäre innerhalb der Einhausung aufweisen.

Häufig wird eine kontrollierte Umgebung, insbesondere eine kontrollierte Atmosphäre, behandelt, um diese Umgebung, insbesondere diese Atmosphäre, in einen Zustand zu bringen, der für eine Behandlung mit der Verarbeitungsanlage oder für eine Behandlung in der Verarbeitungsanlage geeignet ist. Die Behandlung der kontrollierten Umgebung kann beispielsweise eine Bewegung der kontrollierten Umgebung, insbesondere der Atmosphäre, einschließen. Zusätzlich oder alternativ kann die Behandlung eine Reinigung, eine Dekontamination und/oder eine Temperierung der kontrollierten Umgebung, insbesondere der Atmosphäre, einschließen. Darüber hinaus können weitere Behandlungen möglich und wünschenswert sein.

Es hat sich in der Praxis gezeigt, dass die Bearbeitung einer Umgebung häufig unflexibel ist und/oder dass eine vergleichsweise aufwändige Modifikation zumindest eines Teils der Verarbeitungsanlage erforderlich ist, um eine an unterschiedliche Anforderungen angepasste Bearbeitung der Umgebung durchführen zu können.

Es kann wünschenswert sein, diese Nachteile reduzieren oder gar vermeiden zu können.

Der Erfindung liegt die Aufgabe zugrunde, eine Verarbeitungsanlage bereitzustellen, mit welcher die kontrollierte Umgebung in der Verarbeitungsanlage besonders einfach und/oder flexibel behandelt werden kann.

Diese Aufgabe wird durch die Gegenstände mit den Merkmalen der unabhängigen Ansprüche gelöst. Vorteilhafte Ausgestaltungen gehen aus den abhängigen Ansprüchen hervor.

Zur Lösung der gestellten Aufgabe schlägt die Erfindung den Gegenstand mit den Merkmalen von Anspruch 1 vor. Insbesondere wird somit erfindungsgemäß bei einer Verarbeitungsanlage der eingangs beschriebenen Art zur Lösung der genannten Aufgabe vorgeschlagen, dass die Verarbeitungsanlage mindestens eine mit der kontrollierten Umgebung fluidal verbundene Ventilationsmaschine mit einem Stator und einem magnetisch berührungslos gelagerten Rotor aufweist.

Wie eingangs erwähnt, kann die hier beschriebene Verarbeitungsanlage insbesondere eine Verarbeitungsanlage auf der strukturellen und/oder der funktionellen Grundlage eines Isolators, bevorzugt eines Isolators für die pharmazeutische Verarbeitung, sein.

Die Ventilationsmaschine dient der Erzeugung einer Strömung, insbesondere einer Fluidströmung.

Der Stator kann insbesondere ein Antriebsstator sein. Der Stator und der Rotor sind dann derart aufeinander abgestimmt ausgebildet, dass eine vordefinierte Positionierung des Rotors an dem Stator und/oder eine Bewegung des Rotors, insbesondere eine Drehbewegung, erzeugbar ist. Insbesondere können der Stator und der Rotor in der Art von Bestandteilen eines Elektromotors zusammenwirken und/oder eine Bewegung des Rotors kann unter Ausnutzung der Lorenzkraft hervorgerufen werden. Die Ventilationsmaschine kann zu diesem Zweck beispielsweise mindestens eine Leiterspule aufweisen. Zusätzlich oder alternativ kann die Ventilationsmaschine einen magnetischen und/oder einen magnetisierbaren Bestandteil und/oder eine mindestens zweite Leiterspule aufweisen.

Wenn eine Leiterspule von einem elektrischen Strom durchströmt wird, kann ein Feld um diese Leiterspule erzeugt werden, das auf den magnetischen und/oder magnetisierbaren Bestandteil und/oder eine mindestens zweite Leiterspule wirkt. Dadurch kann der magnetische und/oder magnetisierbare Bestandteil und/oder die mindestens zweite Leiterspule angezogen oder abgestoßen werden. Insbesondere kann dadurch eine Drehbewegung erzeugt werden. Zusätzlich oder alternativ kann dadurch beispielsweise die magnetische berührungslose Lagerung des Rotors, insbesondere am Stator, erzielt werden.

Beispielsweise kann der Stator eine als Statorwicklung bezeichnete Leiterspule aufweisen. Der Rotor kann beispielsweise ein magnetisches Material aufweisen. Die Leiterspule und das magnetische Material können miteinander zusammenwirken, wobei ein elektrischer Stromfluss durch die Statorwicklung ein Magnetfeld erzeugen kann, welches das magnetische Material und somit den Rotor in Bewegung versetzt, insbesondere in eine Drehbewegung, und den Rotor in einer vordefinierten Position am Stator hält. Zusätzlich oder alternativ ist es auch möglich, dass der Stator ein magnetisches Material und/oder dass der Rotor eine Leiterspule aufweist.

Insbesondere aufgrund der Bewegung des Rotors, insbesondere aufgrund der Drehbewegung, kann eine Strömung mit der Ventilationsmaschine erzeugt werden. Der Rotor kann zu diesem Zweck bevorzugt ein Rotorblatt oder eine Mehrzahl von Rotorblättern aufweisen. Das Rotorblatt oder die Rotorblätter dienen dazu, eine und insbesondere die hier beschriebene Atmosphäre zu bewegen, wenn der Rotor bewegt wird. Zusätzlich oder alternativ kann der Stator Mittel, beispielsweise Blätter, aufweisen, die in Zusammenwirkung mit der Bewegung des Rotors die oder eine Strömung erzeugen.

Aufgrund der magnetischen berührungslosen Lagerung des Rotors ist es möglich, dass auf Gleitmittel und/oder Schmiermittel in oder an der Lagerung des Rotors verzichtet werden kann. Bei Ventilationsmaschinen mit berührend gelagerten Rotoren sind Gleitmittel und/oder Schmiermittel erforderlich, um eine leichtgängige Bewegung des Rotors zur ermöglichen. In Verarbeitungsanlagen der hier beschriebenen Art ist die magnetische berührungslose Lagerung des Rotors besonders vorteilhaft, weil dadurch Einträge in die kontrollierte Umgebung, insbesondere Einträge in Form von Gleitmittel- und/oder Schmiermittelbestandteilen, reduziert werden können. Darüber hinaus kann es möglich sein, den Rotor auf einfache Weise aus der Ventilationsmaschine zu entnehmen und/oder auszutauschen, so dass eine flexible Modifikation der Ventilationsmaschine und folglich der Verarbeitungsanlage möglich ist.

Der Rotor kann insbesondere umfangsseitig des Stators magnetisch berührungslos gelagert sein. Die Ventilationsmaschine ist dann in der Art eines als Außenläufer bezeichneten Elektromotors ausgebildet. Dadurch kann der Rotor einfach auf den Stator aufsetzbar sein.

Zusätzlich oder alternativ kann die Ventilationsmaschine in der Einhausung, die die kontrollierte Umgebung umgibt, angeordnet sein. Dadurch kann die fluidale Verbindung zwischen der Ventilationsmaschine und der kontrollierten Umgebung als eine unmittelbare Verbindung ausgebildet sein. Dadurch kann die Behandlung der kontrollierten Umgebung mittels der hier beschriebenen Ventilationsmaschine besonders gut planbar und/oder besonders schnell erfolgen. Bevorzugt kann die Ventilationsmaschine in einer Hauptkammer der Einhausung angeordnet sein.

Bei der hier beschriebenen Verarbeitungsanlage kann explizit mehr als eine einzige Ventilationsmaschine vorgesehen sein. Wenn eine Mehrzahl von Ventilationsmaschinen vorgesehen ist, können die Ventilationsmaschinen der Mehrzahl von Ventilationsmaschinen teils unterschiedlich ausgebildet und/oder an unterschiedlichen Positionen der Verarbeitungsanlage angeordnet sein. Es sind insofern mehrere Ventilationsmaschinen mit zumindest einigen der hier beschriebenen Merkmale in einer Verarbeitungsanlage der hier beschriebenen Art kombinierbar.

In der Praxis kann zusätzlich oder alternativ vorgesehen sein, dass die Ventilationsmaschine ein Bestandteil einer Dekontaminationseinrichtung ist.

Die Ventilationsmaschine kann beispielsweise derart ausgebildet sein, dass damit eine Atmosphäre der kontrollierten Umgebung umwälzbar ist und/oder dass eine Dekontaminationssubstanz verteilbar ist. Durch ein Umwälzen der Atmosphäre und/oder ein Verteilen einer Dekontaminationssubstanz kann die kontrollierte Umgebung dekontaminiert werden.

Insbesondere kann die Ventilationsmaschine derart ausgebildet sein, dass die Ventilationsmaschine in einem Betrieb, in welchem mit der Ventilationsmaschine eine Dekontaminationssubstanz verteilt wird, zusätzlich oder alternativ selbst dekontaminiert wird. Dadurch ist eine besonders ausgeprägte Dekontamination erzielbar.

Insbesondere zu diesem Zweck kann die magnetische berührungslose Lagerung des Rotors beispielsweise derart ausgebildet sein, dass ein Spalt zwischen dem Stator und dem an dem Stator gelagerten Rotor vorhanden ist. Der Spalt kann insbesondere mit einer Dekontaminationssubstanz spülbar sein. Beispielsweise kann der Spalt durchströmbar sein. Zusätzlich oder alternativ kann die Ventilationsmaschine Mittel aufweisen, mit welchen zumindest eine Teilströmung der mit der Ventilationsmaschine erzeugten Strömung durch den Spalt führbar ist.

In der Praxis kann weiter zusätzlich oder wieder alternativ vorgesehen sein, dass die Ventilationsmaschine einen Auslass aufweist, aus welchem eine und insbesondere die hier beschriebene Dekontaminationssubstanz in eine und insbesondere die hier beschriebene mit dem Stator und/oder dem Rotor erzeugte Strömung ausführbar ist.

Der Auslass kann insbesondere eine Einstoffdüse oder eine Zweistoffdüse aufweisen oder als eine Einstoffdüse oder eine Zweistoffdüse ausgebildet sein.

Die Dekontaminationssubstanz kann insbesondere Wasserstoffperoxid sein.

Der Auslass kann insbesondere angrenzend an der von der Ventilationsmaschine erzeugten Strömung ausgebildet und/oder angeordnet sein. Wenn der Auslass nahe an dem Rotor und/oder dem Stator ausgebildet ist, kann die Dekontaminationssubstanz besonders effektiv in eine und insbesondere die hier beschriebene, mit dem Stator und/oder dem Rotor erzeugte Strömung ausgeführt werden.

Beispielsweise kann der Auslass oder ein Teil des Auslasses in oder an dem Stator ausgebildet und/oder angeordnet sein. Beispielsweise kann der Auslass an einer Stirnfläche des Stators und/oder an einer transversal zu einer Rotationsachse des Rotors orientierten Fläche des Stators ausgebildet sein. Alternativ kann der Auslass in der Nähe einer in der Verarbeitungsanlage positionierten Medikationsmaschine angeordnet sein.

Es kann auch eine Mehrzahl von Auslässen vorgesehen sein. Die Auslässe der Mehrzahl von Auslässen können insbesondere an den hier beschriebenen Positionen eingerichtet sein. Dadurch kann eine Dekontaminationssubstanz und insbesondere die hier beschriebene Dekontaminationssubstanz besonders schnell und/oder gleichmäßig in der kontrollierten Umgebung verteilt werden.

In der Praxis kann weiter zusätzlich oder wieder alternativ vorgesehen sein, dass der Stator eine geschlossene Oberfläche aufweist. Zusätzlich oder alternativ kann vorgesehen sein, dass der Rotor eine geschlossene Oberfläche aufweist. Insbesondere können der Stator und der Rotor jeweils eine geschlossene Oberfläche aufweisen.

Mit einer geschlossenen Oberfläche ist vorliegend insbesondere gemeint, dass ein Austausch von Partikel von innerhalb des Rotors und/oder Stators mit Partikeln von außerhalb des Rotors und/oder Stators unterbunden ist. Bevorzugt ist die geschlossene Oberfläche auch glatt. Mit einer glatten Oberfläche ist vorliegend insbesondere gemeint, dass keine Unebenheiten, beispielsweise in Form einer Fuge, einer hervorragenden Naht und/oder Stufen, enthalten sind.

Aufgrund der geschlossenen und insbesondere glatten Oberfläche kann die Ablagerung von Verschmutzungen, Partikeln und/oder insbesondere in der Verarbeitungsanlage verarbeiteten pharmazeutischen Erzeugnissen in der Ventilationsmaschine reduziert werden. Insbesondere kann ein Kontaminieren der Ventilationsmaschine reduziert werden. Ferner können Einträge von Partikeln aus der Ventilationsmaschine in die kontrollierte Umgebung reduziert werden.

Die geschlossene und insbesondere glatte Oberfläche des Stators kann beispielsweise erzielt werden, indem der Stator von einer Schicht, insbesondere einer Kunststoffschicht, umgeben ist. Insbesondere kann der Stator eine Einkapselung aus Kunststoff aufweisen. Die geschlossene und insbesondere glatte Oberfläche des Rotors kann beispielsweise erzielt werden, indem der Rotor von einer Schicht, insbesondere einer Kunststoffschicht, umgeben ist. Insbesondere kann der Rotor eine Einkapselung aus Kunststoff aufweisen.

In der Praxis kann weiter zusätzlich oder wieder alternativ vorgesehen sein, dass die Verarbeitungsanlage eine Vibrationseinrichtung zum Vaporisieren einer Dekontaminationssubstanz aufweist. Aufgrund der Vibrationseinrichtung kann die Dekontaminationssubstanz vaporisiert und dann besonders gleichmäßig, gezielt und/oder schnell in der kontrollierten Umgebung verteilt werden.

Die Vibrationseinrichtung zum Vaporisieren einer Dekontaminationssubstanz kann insbesondere eine Ultraschalleinrichtung sein und beispielsweise eine Ultraschallplatte aufweisen. Zum Vaporisieren der Dekontaminationssubstanz kann die Ultraschalleinrichtung im Ultraschall-Frequenzbereich vibrieren. Bei einem Kontakt von flüssiger Dekontaminationssubstanz mit der im Ultraschall-Frequenzbereich vibrierenden Ultraschalleinrichtung wird die Dekontaminationssubstanz aufgrund der hochfrequenten Vibration verdampft oder zunächst in kleine Tröpfchen, insbesondere in ein Aerosol, überführt und anschließend verdampft.

Die Vibrationseinrichtung kann insbesondere in fluidaler Verbindung mit der kontrollierten Umgebung stehen. Insbesondere kann die Vibrationseinrichtung in einer und insbesondere der hier beschriebenen Hauptkammer der Einhausung angeordnet sein. Bevorzugt kann die Vibrationseinrichtung in oder an der Ventilationsmaschine angeordnet sein. Zusätzlich oder alternativ kann die Vibrationseinrichtung in einer Nebenkammer der Verarbeitungsvorrichtung angeordnet sein, welche beispielsweise mittels eines Kanals in fluidaler Verbindung mit einem und insbesondere dem hier beschriebenen Auslass steht.

Optional kann auch eine Vorrichtung zum Ionisieren der vorzugsweise vaporisierten Dekontaminationssubstanz vorgesehen sein. Aufgrund der Ionisation der vorzugsweise vaporisierten Dekontaminationssubstanz kann diese sich bevorzugt an vorbestimmten Oberflächen ablagern und/oder von vorbestimmten Oberflächen abstoßen. Zu diesem Zweck können einige Oberflächen der kontrollierten Umgebung beispielsweise eine positive oder negative elektrische Ladung aufweisen.

In der Praxis kann weiter zusätzlich oder wieder alternativ vorgesehen sein, dass die kontrollierte Umgebung von der Einhausung druckdicht umschließbar ist. Wenn die kontrollierte Umgebung druckdicht umschlossen ist, kann ein Austreten von Partikeln aus der kontrollierten Umgebung in eine die Verarbeitungsvorrichtung umgebende Atmosphäre unterbunden werden. Ebenso kann ein Eintreten von einer die Verarbeitungsvorrichtung umgebenden Atmosphäre in die kontrollierte Umgebung unterbunden werden.

Druckdicht verschließbar bedeutet hier insbesondere, dass ein in der kontrollierten Umgebung, insbesondere in der Atmosphäre der kontrollierten Umgebung, vorliegender Druck gegenüber einem außerhalb der Verarbeitungsvorrichtung vorliegender Druck abgrenzbar ist. Dadurch kann in der Einhausung ein Überdruck oder ein Unterdruck im Vergleich zu einem außerhalb der Einhausung vorliegenden Druck erzeugt werden.

Die Einhausung kann beispielsweise mindestens eine druckdicht verschließbare Tür und/oder mindestens einen druckdicht verschließbaren Flansch aufweisen. Wenn die Verarbeitungsvorrichtung eine Dekontaminationseinrichtung aufweist, kann die mindestens eine Tür und/oder der mindestens eine Flansch während eines Dekontaminationsvorgangs druckdicht verschlossen sein. Wenn der Dekontaminationsvorgang abgeschlossen ist, kann die kontrollierte Umgebung durch die Tür und/oder den Flansch zugänglich sein. Beispielsweise ist es auf diese Weise möglich, eine Substanz, ein Material, eine Probe oder ähnliches, die/das in der kontrollierten Umgebung behandelt wurde, an einen weiteren Verarbeitungsschritt außerhalb der Verarbeitungsanlage zu übergeben.

In der Praxis kann weiter zusätzlich oder wieder alternativ vorgesehen sein, dass die kontrollierte Umgebung eine Reinheit gemäß der Reinraumklasse A aufweist. Die Verarbeitungsvorrichtung und insbesondere die Einhausung kann in vorteilhafter Weise Mittel zum Einstellen einer vorbestimmten Feuchtigkeit, eines vorbestimmten Atmosphärendrucks und/oder einer vorbestimmten Temperatur entsprechend den Anforderungen an die Einhaltung der Reinraumklasse aufweisen.

Mit der Reinraumklasse A ist insbesondere die Reinraumklasse A gemäß den sogenannten Grundsätzen und Leitlinien der Guten Herstellungspraxis der Europäischen Gemeinschaft ("EG-GMP"), Anlage 1, gemeint.

Zusätzlich oder alternativ zu einer Reinheit entsprechend der Reinraumklasse A kann vorgesehen sein, dass die kontrollierte Umgebung eine Reinheit gemäß einer der Reinraumklassen 1 bis 5 gemäß DIN EN ISO 14644-1 aufweist. Zur Einhaltung der Anforderungen dieser Reinraumklasse kann die Verarbeitungsvorrichtung in vorteilhafter Weise entsprechende Mittel aufweisen.

In der Praxis kann weiter zusätzlich oder wieder alternativ vorgesehen sein, dass die Ventilationsmaschine ein Bestandteil einer Reinigungseinrichtung ist. Mit einer Reinigungseinrichtung ist es beispielsweise möglich, größere Partikel, insbesondere Staub, Pulver und/oder Flüssigkeiten, aus der kontrollierten Umgebung zu entfernen.

In der Praxis kann weiter zusätzlich oder wieder alternativ vorgesehen sein, dass die Ventilationsmaschine derart ausgebildet und angeordnet ist, dass eine turbulente Strömung in der kontrollierten Umgebung erzeugbar ist.

Mit einer turbulenten Strömung ist es möglich, eine Atmosphäre in der kontrollierten Umgebung besonders schnell zu durchmischen. Beispielsweise kann auf diese Weise eine und insbesondere die hier beschriebene Dekontaminationssubstanz schnell in der kontrollierten Umgebung verteilt werden. Ebenso ist es möglich, die Dekontaminationssubstanz schnell und/oder vollständig wieder aus der kontrollierten Umgebung zu entfernen. Weiter kann es mit einer turbulenten Strömung besonders einfach möglich sein, eine Reinigung der kontrollierten Umgebung vorzunehmen. Zu diesem Zweck können beispielsweise Partikel von der turbulenten Strömung erfasst und von dieser beispielsweise an eine vorbestimmte Position in der Einhausung transportiert werden. Die Partikel können an der vorbestimmten Position beispielsweise abgesaugt werden. Hierfür kann die Reinigungseinrichtung oder die Verarbeitungsanlage eine Absaugvorrichtung aufweisen.

In der Praxis kann weiter zusätzlich oder wieder alternativ vorgesehen sein, dass Leitflächen zur Führung einer und insbesondere der hier beschriebenen Strömung ausgebildet sind.

Mit den Leitflächen kann die erzeugte Strömung in eine vorbestimmte Richtung lenkbar sein. Die Leiflächen können zusätzlich oder alternativ verstellbar sein. Mit lenkbaren Leitflächen ist/sind das Umwälzen der Atmosphäre der kontrollierten Umgebung und/oder das Reinigen der kontrollierten Umgebung besonders effektiv durchführbar.

Die Leitflächen sind insbesondere stromabwärts an der erzeugten Strömung angrenzend angeordnet. Beispielsweise können die Leitflächen an dem Stator und/oder an einer Wandung der Einhausung angeordnet sein. Weiter zusätzlich oder wieder alternativ können die Leitflächen Bestandteile eines Gehäuses der Ventilationsmaschine sein.

In der Praxis kann weiter zusätzlich oder wieder alternativ vorgesehen sein, dass die Verarbeitungsanlage eine Filtervorrichtung zum Filtern von Partikeln aufweist. Die Filtervorrichtung kann insbesondere einen Atmosphärenfilter und bevorzugt einen Luftfilter aufweisen. Die Filtervorrichtung kann so ausgebildet und angeordnet sein, dass während eines Umwälzens einer Atmosphäre der kontrollierten Teilchen aus der Atmosphäre herausfilterbar sind. Insbesondere kann die Filtervorrichtung so ausgebildet sein, dass ausschließlich gasförmige Teilchen und/oder Partikel bis zu einer vorbestimmten Größe durch die Filtervorrichtung transportierbar sind.

Die Filtervorrichtung zum Filtern von Partikeln kann beispielsweise dazu beitragen, dass die kontrollierte Umgebung eine Reinheit entsprechend der Reinraumklasse A oder eine Reinheit gemäß einer der Reinraumklassen 1 bis 5 gemäß DIN EN ISO 14644-1 aufweist.

In der Praxis kann weiter zusätzlich oder wieder alternativ vorgesehen sein, dass der Stator und/oder der Rotor derart ausgebildet ist/sind, dass eine und insbesondere die hier beschriebene Strömung zumindest anteilig radial von der Ventilationsmaschine abgeblasen wird. Die Ventilationsmaschine kann in diesem Fall beispielsweise in der Art eines Diagonalventilators ausgebildet sein.

Insbesondere kann vorgesehen sein, dass der Stator und/oder der Rotor derart ausgebildet ist/sind, dass die Strömung vollständig radial von der Ventilationsmaschine abgeblasen wird. Die Ventilationsmaschine kann in diesem Fall beispielsweise in der Art eines Radialventilators ausgebildet sein. Bei einem Radialventilator wird die Atmosphäre im Wesentlichen parallel zu der Rotationsachse des Rotors angesaugt und durch eine Bewegung des spezifisch ausgebildeten Rotors umgelenkt und radial ausgeblasen.

Mit einer radial abgeblasenen Strömung ist es beispielsweise möglich, die Atmosphäre der kontrollierten Umgebung besonders homogen umzuwälzen.

Ein Rotor, mit dem die Strömung anteilig und insbesondere vollständig radial abgeblasen wird, kann insbesondere derart ausgebildet sein, dass der Rotor in einer beliebigen Orientierung an dem Stator anordbar ist. Beispielsweise kann der Rotor symmetrisch bezüglich einer orthogonal zu der Rotationsachse des Rotors angeordneten Ebene ausgebildet sein.

In der Praxis kann weiter zusätzlich oder wieder alternativ vorgesehen sein, dass der Stator und/oder der Rotor derart ausgebildet ist/sind, dass eine Strömung zumindest anteilig axial von der Ventilationsmaschine abgeblasen wird. Die Ventilationsmaschine kann in diesem Fall beispielsweise in der Art eines Diagonalventilators ausgebildet sein.

Insbesondere kann vorgesehen sein, dass der Stator und/oder der Rotor derart ausgebildet ist/sind, dass die Strömung vollständig axial von der Ventilationsmaschine abgeblasen wird. Die Ventilationsmaschine kann in diesem Fall beispielsweise in der Art eines Axialventilators ausgebildet sein. Bei einem Axialventilator wird die Atmosphäre im Wesentlichen parallel zu der Rotationsachse des Rotors angesaugt und durch eine Bewegung des spezifisch ausgebildeten Rotors parallel zu der Rotationsachse des Rotors ausgeblasen.

Mit einer axial abgeblasenen Strömung ist es beispielsweise möglich, Partikel in der kontrollierten Umgebung besonders einfach zu bewegen und/oder die kontrollierte Umgebung besonders gründlich zu reinigen.

In der Praxis kann weiter zusätzlich oder wieder alternativ vorgesehen sein, dass der Rotor derart ausgebildet ist, dass mit einer Drehung in einer ersten Drehrichtung eine zumindest anteilig radial von der Ventilationsmaschine abgeblasene Strömung erzeugbar ist und dass mit einer Drehung in einer zweiten Drehrichtung eine zumindest anteilig axial von der Ventilationsmaschine abgeblasene Strömung erzeugbar ist. Aufgrund der magnetischen berührungslosen Lagerung kann die Drehrichtung des Rotors besonders einfach und/oder schnell gewechselt werden. Es ist lediglich erforderlich ein mit der Leiterspule erzeugtes Magnetfeld entsprechend zu steuern.

Wenn der Rotor die hier beschriebe Ausbildung aufweist, kann der einfache und/oder schnelle Wechsel der Drehrichtung ausgenutzt werden, um unterschiedliche Strömungen mit einer einzigen Ventilationsmaschine zu erzeugen, nämlich insbesondere eine im Wesentlichen radiale Strömung und eine im Wesentlichen axiale Strömung. Hinsichtlich der mit diesen beiden Strömungen verbundenen Vorteile wird auf die

Beschreibung dieser Strömungen verwiesen.

In der Praxis kann weiter zusätzlich oder wieder alternativ vorgesehen sein, dass der Stator und der Rotor derart ausgebildet sind, dass diese beiden Bauteile in einer einzigen Orientierung zueinander aneinander anordbar sind. Dadurch ist es möglich, ein Anordnen des Rotors an dem Stator in einer falschen Orientierung, die beispielsweise von einer Strömung in eine nicht gewünschte Orientierung begleitet sein kann, zu unterbinden. Diese Ausbildung kann insbesondere in Verbindung mit einer Ausbildung des Stators und/oder des Rotors, aufgrund welcher eine Strömung zumindest anteilig axial von der Ventilationsmaschine abgeblasen wird, vorteilhaft sein.

In der Praxis kann weiter zusätzlich oder wieder alternativ vorgesehen sein, dass die Stator-und-Rotor-Kombination eine geometrische Codierung hat. Dadurch kann beispielsweise sichergestellt werden, dass der Stator und der Rotor in einer einzigen Orientierung zueinander aneinander anordbar sind.

Insbesondere kann die Stator-und-Rotor-Kombination einen kegelstumpfförmig ausgebildeten Abschnitt und eine dazu komplementäre Gegenform aufweisen. Zusätzlich oder alternativ kann die Stator-und-Rotor-Kombination einen gestuften Absatz und eine dazu komplementäre Gegenform aufweisen. Die genaue Verortung der hier beschriebenen Abschnitte, Absätze und/oder Gegenformen an dem Stator und/oder dem Rotor kann insbesondere von der übrigen Ausgestaltung des Stators und des Rotors abhängen. Insbesondere, wenn die Ventilationsmaschine in der beschriebenen Art eines Außenläufers ausgebildet ist, kann der Stator den kegelstumpfförmig ausgebildeten Abschnitt und/oder den gestuften Absatz aufweisen und der Rotor kann in einem Mittelteil eine dazu komplementäre Gegenform aufweisen.

In der Praxis kann weiter zusätzlich oder wieder alternativ vorgesehen sein, dass die Verarbeitungsanlage eine Beheizungseinrichtung aufweist. Mit der Beheizungseinrichtung ist es möglich, eine vordefinierte Temperatur in der Verarbeitungsanlage einzustellen. Eine mit der Beheizungseinrichtung eingestellte, vordefinierte Temperatur kann für die Verarbeitung einer Substanz, eines Material, einer Probe oder ähnlichem in der kontrollierten Umgebung vorteilhaft sein. Insbesondere kann durch eine mit der Beheizungseinrichtung eingestellten Temperatur auch eine Feuchtigkeit in der kontrollierten Umgebung vordefiniert beeinflusst werden. Denn die in einer Atmosphäre maximal enthaltbare Menge Wasser ist von der Temperatur der Atmosphäre abhängig.

Zusätzlich oder alternativ kann vorgesehen sein, dass die Ventilationsmaschine eine und insbesondere die hier beschriebene Beheizungseinrichtung aufweist. Dadurch kann die umgewälzte Atmosphäre besonders direkt und homogen beheizt werden.

Die Beheizungseinrichtung kann insbesondere als eine elektrisch betriebene und weiter insbesondere auf einem Widerstand oder einer Induktion basierende Beheizungsvorrichtung ausgebildet sein. Zusätzlich oder alternativ kann die Beheizungsvorrichtung in dem Rotor und/oder in dem Stator ausgebildet sein. Dadurch werden der Rotor und/oder der Stator erwärmt und der Rotor und/oder Stator kann/können die Atmosphäre unmittelbar während des Umwälzens erwärmen.

In der Praxis kann weiter zusätzlich oder wieder alternativ vorgesehen sein, dass der Stator als ein in die kontrollierte Umgebung hineinragender Bestandteil der Einhausung ausgebildet ist. Zu diesem Zweck können der Stator und die Einhausung einstückig ausgebildet sein. Alternativ können der Stator und der Rotor als separate Bauteile ausgebildet sein, die miteinander mechanisch miteinander verbunden oder verbindbar sind. Dadurch können Spalte und/oder geometrische Hinterschneidungen verringert sein und/oder eine Ablagerung von Verunreinigungen reduziert sein.

Insbesondere können die Einhausung und der von der Einhausung in die kontrollierte Umgebung hineinragende Stator zumindest an der Grenze zwischen dem Stator und dem angrenzenden Bestandteil der Einhausung eine geschlossene und insbesondere glatte Oberfläche ausbilden. Für die in Zusammenhang mit der vorliegenden Erfindung verbundenen Vorteile einer geschlossenen und insbesondere glatten Oberfläche wird auf die Vorteile der geschlossenen und insbesondere glatten Oberfläche des Rotors und/oder Stators verwiesen.

In der Praxis kann weiter zusätzlich oder wieder alternativ vorgesehen sein, dass die Ventilationsmaschine ein und insbesondere das hier beschriebene Gehäuse aufweist. Mit dem Gehäuse kann es möglich sein, eine mit dem Rotor und/oder dem Stator erzeugte Strömung umzulenken und/oder gerichtet abzugeben, beispielsweise für die hier beschriebene Reinigung der kontrollierten Umgebung. Zudem kann das Gehäuse in vorteilhafter Weise dazu geeignet sein, eine Verunreinigung und/oder eine Kontamination der Ventilationsmaschine zu reduzieren oder sogar zu vermeiden. Insbesondere kann das Gehäuse mittels eines Verschlussstücks bedarfsweise öffenbar und schließbar ausgebildet sein.

In der Praxis kann weiter zusätzlich oder wieder alternativ vorgesehen sein, dass die Einhausung eine Hauptkammer und mindestens eine fluidal abtrennbare Nebenkammer aufweist, wobei die Ventilationsmaschine in der Nebenkammer angeordnet ist. Wenn die Ventilationsmaschine in der Nebenkammer angeordnet ist, können die in Verbindung mit der Ventilationsmaschine beschriebenen Wirkungen insbesondere in der Nebenkammer erzielt werden.

Die Hauptkammer kann beispielsweise eine Kammer der Verarbeitungsanlage sein, in welcher eine Verarbeitung einer Substanz, eines Materials, einer Probe oder ähnlichem erfolgt. Die Nebenkammer kann insbesondere eine Zuluftleitung, eine Abluftleitung und/oder eine Schleusenkammer sein, die fluidal mit der Hauptkammer verbindbar ist.

Wenn die Ventilationsmaschine in einer Zuluftleitung, einer Abluftleitung und/oder einer Schleusenkammer angeordnet ist, kann eine kontrollierte Umgebung, insbesondere eine Atmosphäre, in der Zuluftleitung, der Abluftleitung und/oder der Schleusenkammer bereits an eine kontrollierte Umgebung, insbesondere eine Atmosphäre, in der Hauptkammer angenähert werden, bevor die Nebenkammer und die Hauptkammer miteinander fluidal verbunden werden oder sind. So kann ein unerwünschter Eintrag von Partikeln, Teilchen und/oder Substanzen in die Hauptkammer reduziert oder verhindert werden.

In der Praxis kann weiter zusätzlich oder wieder alternativ vorgesehen sein, dass die Ventilationsmaschine ortsveränderlich angeordnet ist. Mit anderen Worten kann die Verarbeitungsanlage und/oder die Ventilationsmaschine derart ausgebildet sein, dass die Ventilationsmaschine in Relation zu der Verarbeitungsanlage, insbesondere innerhalb der Einhausung, bewegt werden kann. Dadurch kann es möglich sein, Bereiche der kontrollierten Umgebung bei Bedarf selektiv zu behandeln und/oder verstärkt zu behandeln als andere Bereiche der kontrollierten Umgebung.

In der Praxis kann weiter zusätzlich oder wieder alternativ vorgesehen sein, dass die Ventilationsmaschine auf einem magnetischen Fördersystem angeordnet ist. Insbesondere kann die Ventilationsmaschine auf einem Magnetmover des magnetischen Fördersystems angeordnet sein.

Ein magnetisches Fördersystem kann insbesondere dazu eingerichtet sein, kleine bis mittlere Lasten zu transportieren. Es umfasst ein bevorzugt modular ausgebildetes Leitsystem und mindestens einen Magnetmover. Das Leitsystem wird auch als Magnetfelderzeuger bezeichnet. Es kann insbesondere als ein Planarmotor oder als ein Bestandteil eines Planarmotors ausgebildet sein. Der Magnetmover ist eine Transportplattform, die in oder auf dem Magnetfelderzeuger levitiert und/oder transportiert werden kann.

Der Antrieb des Magnetmovers kann insbesondere durch eine magnetische Wechselwirkung zwischen dem Magnetfelderzeuger und der Magnetmover erzeugt werden. Der Magnetmover kann aufgrund der magnetischen Wechselwirkung in der Schwebe gehalten und schwebend von einer und insbesondere der beschriebenen magnetischen Wechselwirkung in dem Magnetfelderzeuger geführt werden.

Mit der Anordnung der Ventilationsmaschine auf dem magnetischen Fördersystem, insbesondere dem Magnetmover, kann die Ventilationsmaschine besonders flexibel und/oder widerstandsarm transportiert werden. Ferner kann das magnetische Fördersystem, insbesondere der Magnetmover, im Vergleich zu anderen Fördersystemen in vorteilhafter Weise einen stark begrenzten Eintrag von Verunreinigungen in die kontrollierte Umgebung aufweisen.

In der Praxis kann weiter zusätzlich oder wieder alternativ vorgesehen sein, dass die Ventilationsmaschine an einem bewegbaren Roboterarm angeordnet ist. Der Roboterarm kann dazu dienen, die Ventilationsmaschine zu bewegen. Der Roboterarm kann beispielsweise ein Cobot sein. Zusätzlich oder alternativ kann der Roboterarm an einer Wandung der Einhausung angeordnet sein. Wieder zusätzlich oder alternativ kann der Roboterarm an einer Transportplattform insbesondere dem und bevorzugt dem hier beschriebenen Magnetmover des magnetischen Fördersystems angeordnet sein.

Mit der Anordnung der Ventilationsmaschine an dem Roboterarm kann eine besonders hohe Flexibilität bei einer Positionierung der Ventilationsmaschine in der kontrollierten Umgebung erzielt werden.

In der Praxis kann weiter zusätzlich oder wieder alternativ vorgesehen sein, dass die Verarbeitungsanlage einen Energiespeicher zur Versorgung der Ventilationsmaschine mit elektrischer Energie aufweist. Die Ventilationsmaschine kann dann energetisch autark von einer externen Energieversorgung betreibbar sein. Die elektrische Energie kann insbesondere dazu dienen, dass der Rotor magnetisch gelagert und/oder bewegt werden kann.

Insbesondere kann die Ventilationsmaschine den Energiespeicher zur Versorgung der Ventilationsmaschine mit elektrischer Energie aufweisen. Mit anderen Worten ausgedrückt, kann der Energiespeicher in oder an der Ventilationsmaschine angeordnet sein. Insbesondere kann der Energiespeicher in dem Stator oder in dem Rotor angeordnet sein. Es können auch zwei Energiespeicher oder Teile eines Energiespeichers jeweils in dem Stator und dem Rotor angeordnet sein. Zusätzlich oder alternativ kann der Energiespeicher in einem separaten Bauteil angeordnet sein, das bedarfsweise mit Stator und/oder dem Rotor verbindbar oder verbunden ist. Diese Ausführung des Energiespeichers kann insbesondere im Zusammenhang mit einer ortsveränderlich angeordneten Ventilationsmaschine vorteilhaft sein, weil dann auf das Führen von Kabeln in der kontrollierten Umgebung zum Versorgen der Ventilationsmaschine mit elektrischer Energie verzichtet werden kann.

Der Energiespeicher kann insbesondere ein Akkumulator sein.

In der Praxis kann weiter zusätzlich oder wieder alternativ vorgesehen sein, dass der Stator eingekapselt ausgebildet ist. Beispielsweise kann der Stator von einer unmittelbar an der Oberfläche des Stators anliegenden Schicht umgeben sein. Der einkapselte Stator kann zusätzlich oder alternativ einen Spalttopf aufweisen, welcher beispielsweise geschlossen ausgebildet ist und/oder von einer zusätzlichen Einkapselung mit einer geschlossenen Oberfläche umgeben ist. Alternativ kann der beispielsweise den Spalttopf aufweisende Stator mit einer Abdeckung abgedeckt sein, wobei die Abdeckung eine geschlossen ausgebildete Oberfläche mit der Einhausung ausgebildet.

Zusätzlich oder alternativ kann vorgesehen sein, dass der Rotor eingekapselt ausgebildet ist. Beispielsweise kann der Rotor von einer unmittelbar an der Oberfläche des Rotors anliegenden Schicht umgeben sein.

Wieder zusätzlich oder alternativ kann vorgesehen sein, dass der Energiespeicher eingekapselt ausgebildet ist.

Mit der Einkapselung kann eine geschlossene und insbesondere glatte Oberfläche erzielt werden. Es wird auf die in diesem Zusammenhang beschriebenen Vorteile verwiesen.

Die Einkapselung kann beispielsweise aus Kunststoff ausgebildet sein. Sie kann als Beschichtung aufgebracht oder als eine elastischer Schicht aufgezogen sein.

Die Einkapselung kann insbesondere in Kombination mit der Anordnung eines und bevorzugt des hier beschriebenen Energiespeichers in dem Rotor, dem Stator und/oder einem separaten Bauteil vorteilhaft sein. Durch die Einkapselung kann effektiv verhindert werden, dass Partikel, Teilchen oder ähnliches aus dem Energiespeicher in die kontrollierte Umgebung eingetragen werden.

In der Praxis kann weiter zusätzlich oder wieder alternativ vorgesehen sein, dass mindestens ein Bereich der Verarbeitungsanlage eine katalytische Beschichtung aufweist. Die katalytische Beschichtung kann insbesondere auf eine vordefinierte, in der kontrollierten Umgebung durchzuführende Reaktion funktional abgestimmt sein.

Der mit der katalytischen Beschichtung beschichtete Bereich kann insbesondere mit der umgewälzten Atmosphäre in fluidalem Kontakt stehen. Wenn die umgewälzte Atmosphäre und/oder ein/eine in der Atmosphäre enthaltene/r Substanz und/oder Stoff mit der katalytischen Beschichtung in Kontakt gelangen, kann in vorteilhafter Weise eine Reaktionsgeschwindigkeit einer in der kontrollierten Umgebung durchzuführenden insbesondere chemischen Reaktion erhöht werden.

Insbesondere kann mindestens ein Bereich der Ventilationsmaschine eine katalytische Beschichtung aufweisen. Bevorzugt kann der Rotor und/oder eine Einkapselung des Rotors die katalytische Beschichtung aufweisen.

Aufgrund der Beschichtung eines Bereichs der Ventilationsmaschine, insbesondere des Rotors, kann es möglich sein, dass die chemische Reaktion unmittelbar an und/oder in der Ventilationsmaschine stattfindet. Insbesondere ein hoher Anteil der zu reagierenden Stoffe bereits in oder an der Ventilationsmaschine reagiert.

In der Praxis kann weiter zusätzlich oder wieder alternativ vorgesehen sein, dass die katalytische Beschichtung ein Manganoxid aufweist. Zusätzlich oder alternativ kann die katalytische Beschichtung ein Manganoxid sein. Die Verwendung von Manganoxid ist insbesondere vorteilhaft, wenn eine Dekontaminationssubstanz, insbesondere Wasserstoffperoxid, für eine Dekontamination der kontrollierten Umgebung verwendet wird. Das Manganoxid kann dazu dienen, das Wasserstoffperoxid chemisch aufzuspalten.

Zur Lösung der gestellten Aufgabe schlägt die Erfindung alternativ die Merkmale des nebengeordneten Verfahrensanspruchs vor. Insbesondere wird somit erfindungsgemäß bei einem Verfahren der eingangs beschriebenen Art zur Lösung der genannten Aufgabe vorgeschlagen, dass mittels der Ventilationsmaschine eine Atmosphäre in der kontrollierten Umgebung bewegt wird.

Durch ein Bewegen, insbesondere ein Umwälzen, der Atmosphäre in der kontrollierten Umgebung kann die kontrollierte Umgebung besonders flexibel und einfach bearbeitet werden.

Es wird explizit erwähnt, dass die mit der Umwälzung der Atmosphäre in Zusammenhang mit der Verarbeitungsanlage beschriebenen Merkmale und damit verbundenen Vorteile analog auf das Verfahren übertragbar sind.

In der Praxis kann zusätzlich oder alternativ vorgesehen sein, dass die Ventilationsmaschine händisch durch eine Öffnung in einer und insbesondere in der hier beschriebenen Einhausung der Verarbeitungsanlage bestimmungsgemäß positioniert wird.

Zusätzlich oder alternativ kann vorgesehen sein, dass zumindest der Rotor der Ventilationsmaschine händisch durch eine Öffnung in einer und insbesondere in der hier beschriebenen Einhausung der Verarbeitungsanlage bestimmungsgemäß positioniert wird.

Wieder zusätzlich oder alternativ kann vorgesehen sein, dass die Ventilationsmaschine oder zumindest der Rotor der Ventilationsmaschine händisch durch einen Handschuheingriff der Verarbeitungsanlage bestimmungsgemäß positioniert wird.

Wieder zusätzlich oder alternativ kann vorgesehen sein, dass die Ventilationsmaschine oder zumindest der Rotor der Ventilationsmaschine mit einem magnetischen Fördersystem, insbesondere einem Magnetmover eines magnetischen Fördersystems, bestimmungsgemäß positioniert wird. Dies kann insbesondere durch eine Öffnung in einer und insbesondere in der hier beschriebenen Einhausung der Verarbeitungsanlage erfolgen.

Wieder zusätzlich oder alternativ kann vorgesehen sein, dass die Ventilationsmaschine oder zumindest der Rotor der Ventilationsmaschine mit einem bewegbaren Roboterarm bestimmungsgemäß positioniert wird. Dies kann insbesondere durch eine Öffnung in einer und insbesondere in der hier beschriebenen Einhausung der Verarbeitungsanlage erfolgen.

Die hier beschriebenen Arten der Positionierung der Ventilationsmaschine oder zumindest des Rotors kann insbesondere durch eine vor und/oder hinter der Öffnung oder dem Handschuheingriff angeordnete Schleusenkammer erfolgen.

In der Praxis kann weiter zusätzlich oder wieder alternativ vorgesehen sein, dass die Ventilationsmaschine nach der Einnahme der bestimmungsgemäßen Position für eine vorbestimmte Dauer ruht, bevor die Atmosphäre in der kontrollierten Umgebung bewegt wird. Durch das Ruhen der Ventilationsmaschine kann eine durch das Positionieren der Ventilationsmaschine bewegte Atmosphäre zur Ruhe kommen.

In der Praxis kann weiter zusätzlich oder wieder alternativ vorgesehen sein, dass mittels der Ventilationsmaschine die kontrollierte Umgebung gereinigt wird.

Insbesondere kann bei der Reinigung der kontrollierten Umgebung mittels der Ventilationsmaschine eine turbulente Strömung erzeugt werden.

Die turbulente Strömung kann insbesondere mit Leitflächen geleitet und insbesondere in der kontrollierten Umgebung geführt werden. Die turbulente Strömung kann beispielsweise dazu verwendet werden, Partikel, insbesondere Staub, Pulver und/oder Flüssigkeiten, zu erfassen, zu bewegen und/oder aus der kontrollierten Umgebung zu entfernen, wie im Zusammenhang mit der Beschreibung der Verarbeitungsanlage beschrieben.

In der Praxis kann weiter zusätzlich oder wieder alternativ vorgesehen sein, dass eine Flüssigkeit auf Wasserbasis in die kontrollierte Umgebung eingebracht wird und die Flüssigkeit auf Wasserbasis mittels der Ventilationsmaschine verteilt wird.

Die Flüssigkeit auf Wasserbasis kann insbesondere Wasser oder eine wässrige Lösung sein. Zusätzlich oder alternativ kann die Flüssigkeit auf Wasserbasis Wasser und mindestens eine Detergens, beispielsweise Laugenseife und/oder Alkohol, enthalten. Ein Detergens setzt die Grenzflächenspannung zwischen einer zu reinigenden Oberfläche, eines darauf befindlichen Schmutzes und eines Lösemittels, insbesondere des Wassers, herab. Dadurch ist eine Reinigung einer zu reinigenden Oberfläche in der kontrollierten Umgebung besonders einfach.

Die Flüssigkeit auf Wasserbasis kann insbesondere in die Ventilationsmaschine eingebracht werden, vorzugsweise durch einen und insbesondere den hier beschriebenen Auslass.

Mit der Flüssigkeit auf Wasserbasis kann die kontrollierte Umgebung beispielsweise gereinigt werden.

In der Praxis kann weiter zusätzlich oder wieder alternativ vorgesehen sein, dass mittels der Ventilationsmaschine eine vorbestimmte Feuchtigkeit in der kontrollierten Umgebung erzeugt wird.

Die vorbestimmte Feuchtigkeit kann insbesondere ein Wassergehalt in der Atmosphäre der kontrollierten Umgebung von 15 % bis 30 %, bevorzugt 20 % bis 25 %, sein. Mit einer vorbestimmten, insbesondere niedrigen, Feuchtigkeit kann eine Dekontaminationssubstanz, insbesondere Wasserstoffperoxid, besonders effektiv zur Dekontamination der kontrollierten Umgebung verwendet werden. Das Erzeugen einer vorbestimmten Feuchtigkeit kann insbesondere vorteilhaft sein, nachdem die kontrollierte Umgebung unter Verwendung einer Flüssigkeit auf Wasserbasis gereinigt worden ist.

In der Praxis kann weiter zusätzlich oder wieder alternativ vorgesehen sein, dass mittels der Ventilationsmaschine die kontrollierte Umgebung dekontaminiert wird. Insbesondere kann eine Dekontaminationssubstanz in die kontrollierte Umgebung eingebracht und diese mittels der Ventilationsmaschine verteilt werden.

Die Dekontaminationssubstanz kann insbesondere Wasserstoffperoxid sein.

Eine Dekontamination der kontrollierten Umgebung kann wie beschrieben besonders effektiv sein, wenn zuvor eine vorbestimmte, insbesondere niedrige, Feuchtigkeit in der Atmosphäre der kontrollierten Umgebung erzeugt wird.

Die Dekontaminationssubstanz kann insbesondere eingebracht werden, wenn die Ventilationsmaschine ruht. Zusätzlich kann ein Starten der Ventilationsmaschine und insbesondere ein Erzeugen einer Strömung mit dem Rotor und/oder dem Stator für eine vorbestimmte Dauer nach dem Einbringen der Dekontaminationssubstanz verzögert sein.

In der Praxis kann weiter zusätzlich oder wieder alternativ vorgesehen sein, dass die Dekontaminationssubstanz in flüssiger Form eingebracht und vor dem Verteilen vaporisiert wird.

Das Vaporisieren kann beispielsweise durch eine Ultraschallbehandlung der flüssigen Dekontaminationssubstanz herbeigeführt werden. Es ist auch möglich, dass die Dekontaminationssubstanz versprüht wird. Zusätzlich oder alternativ kann das Vaporisieren durch ein Erhitzen der flüssigen Dekontaminationssubstanz herbeigeführt werden.

Das Vaporisieren der Dekontaminationssubstanz kann insbesondere vorteilhaft sein, wenn die Ventilationsmaschine ruht. Bevorzugt wird die Ventilationsmaschine gestartet, wenn eine vorbestimmte Menge der Dekontaminationssubstanz vaporisiert oder versprüht ist.

Insbesondere kann die Dekontaminationssubstanz auch ionisiert werden. Mit Ionisation ist ein Aufbringen einer elektrischen Ladung auf die vaporisierte oder versprühte Dekontaminationssubstanz gemeint. Die ionisierte, vaporisierte und/oder versprühte Dekontaminationssubstanz kann sich besonders gut auf vorbestimmten, insbesondere elektrisch entgegengesetzt geladenen, Oberflächen ansiedeln. Zusätzlich oder alternativ ist es möglich, dass die ionisierte, vaporisierte und/oder versprühte Dekontaminationssubstanz von insbesondere elektrisch gleichartig geladenen Oberflächen abgestoßen werden.

In der Praxis kann weiter zusätzlich oder wieder alternativ vorgesehen sein, dass die Drehrichtung des Rotors gewechselt wird. Insbesondere kann die Drehrichtung des Rotors wiederkehrend gewechselt werden. Der Roter kann wie beschrieben zu einer Erzeugung einer Strömung dienen. Wenn die Drehrichtung insbesondere wiederkehrend gewechselt wird, kann auf einfache Weise eine turbulente und insbesondere großvolumige Strömung erzeugt werden. So kann eine Atmosphäre in der kontrollierten Umgebung besonders einfach und/oder schnell umgewälzt werden.

Es ist auch möglich, den Rotor derart zu steuern, dass eine Drehrichtung in einer frühen Arbeitsphase konstant ist und in einer späten Arbeitsphase wiederkehrend wechselt. In der frühen Arbeitsphase kann dann beispielsweise eine Strömung mit hohem Druck erzeugt werden, so dass eine Reinigung der kontrollierten Umgebung durchgeführt werden kann. In der späten Arbeitsphase kann dann die beschriebene Umwälzung der Atmosphäre erfolgen, beispielsweise um eine Dekontaminationssubstanz möglichst schnell und/oder homogen zu verteilen.

In der Praxis kann weiter zusätzlich oder wieder alternativ vorgesehen sein, dass nach dem Verteilen der Dekontaminationssubstanz eine reine Atmosphäre mit der Ventilationsmaschine verteilt wird. Insbesondere kann die reine Atmosphäre mit der Ventilationsmaschine verteilt werden, um die kontrollierte Umgebung zu spülen. Unter einer reinen Atmosphäre ist insbesondere eine Atmosphäre zu verstehen, die frei von einer Dekontaminationssubstanz ist, beispielsweise eine Atmosphäre, die hauptsächlich Stickstoff und bevorzugt Luft enthält.

Die Erfindung wird nachfolgend anhand einiger Ausführungsbeispiele näher beschrieben, ist jedoch nicht auf diese Ausführungsbeispiele beschränkt. Weitere Erfindungsvarianten und Ausführungsbeispiele ergeben sich durch Kombination der Merkmale einzelner oder mehrerer Schutzansprüche untereinander und/oder mit einzelnen oder mehreren Merkmalen der Ausführungsbeispiele und/oder der zuvor beschriebenen Varianten erfindungsgemäßer Vorrichtungen und Verwendungen.

Es zeigt:
Fig. 1 eine schematische Darstellung einer hier beschriebenen Verarbeitungsanlage mit einer kontrollierten Umgebung und einer Mehrzahl von Ventilationsmaschinen,
Fig. 2 eine schematische Darstellung einer hier beschriebenen Verarbeitungsanlage mit einer kontrollierten Umgebung und einer Ventilationsmaschine,
Fig. 3 eine detailliere Ansicht einer Ventilationsmaschine in einem Längsschnitt,
Fig. 4 eine weitere schematische Darstellung einer hier beschriebenen Verarbeitungsanlage mit einer kontrollierten Umgebung und einer Ventilationsmaschine,
Fig. 5 eine noch weitere schematische Darstellung einer hier beschriebenen Verarbeitungsanlage mit einer kontrollierten Umgebung und einer Ventilationsmaschine,
Fig. 6 eine schematische Darstellung einer hier beschriebenen Verarbeitungsanlage mit einer kontrollierten Umgebung und zwei eingehausten Ventilationsmaschinen,
Fig. 7 eine schematische Darstellung einer hier beschriebenen Verarbeitungsanlage mit einer kontrollierten Umgebung, einem Ventilationsmaschine und einer Schleusenkammer,
Fig. 8 eine schematische Darstellung einer hier beschriebenen Verarbeitungsanlage mit einer kontrollierten Umgebung und einer auf einem magnetischen Fördersystem angeordneten Ventilationsmaschine,
Fig. 9 eine schematische Darstellung einer hier beschriebenen Verarbeitungsanlage mit einer kontrollierten Umgebung und einer auf einem Roboterarm angeordneten Ventilationsmaschine, und
Fig. 10 eine schematische Darstellung eines Verfahrens zum Behandeln einer kontrollierten Umgebung der Verarbeitungsanalage mit einer Ventilationsmaschine.

Bei der nachfolgenden Beschreibung verschiedener Gegenstände und Ausführungsbeispiele der Erfindung erhalten in ihrer Funktion übereinstimmende Elemente auch bei abweichender Gestaltung oder Formgebung übereinstimmende Bezugszeichen. Zur besseren Übersicht sind in den Figuren nicht alle Bezugszeichen gesetzt, obwohl die Elemente sehr wohl in den Figuren vorhanden sein können.

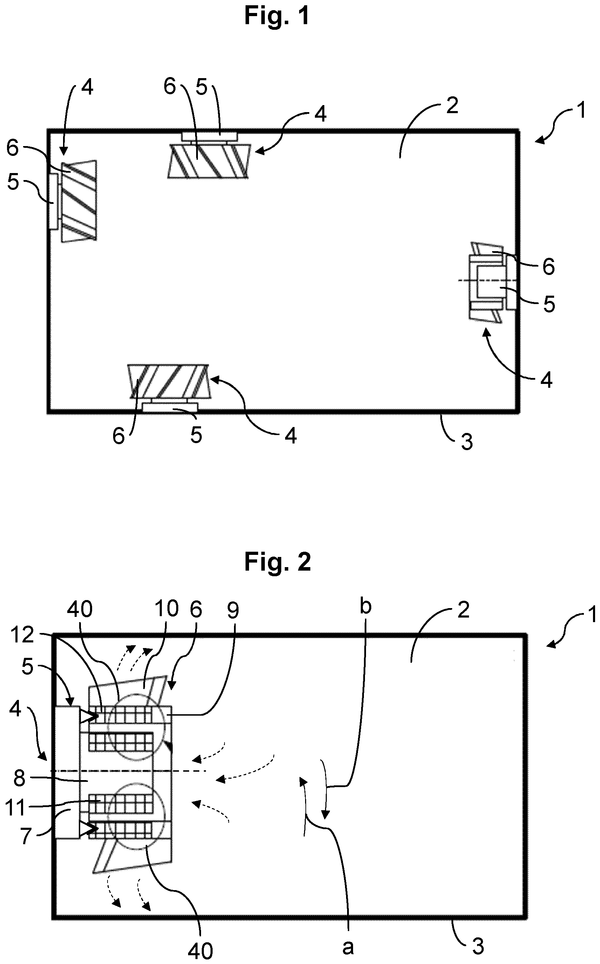

Die in Figur 1 gezeigte schematisch dargestellte Verarbeitungsanlage 1 weist eine kontrollierte Umgebung 2 und die kontrollierte Umgebung 2 umgebenden Einhausung 3 auf. Ferner weist die Verarbeitungsanlage 1 mindestens eine mit der kontrollierten Umgebung 2 fluidal verbundene Ventilationsmaschine 4, nämlich vier Ventilationsmaschinen, auf. Die Ventilationsmaschinen 4 sind unmittelbar in der kontrollierten Umgebung 2 angeordnet. Drei der vier Ventilationsmaschinen 4 sind in einer Ansicht von der Seite gezeigt. Die vierte Ventilationsmaschine 4 ist in einem Längsschnitt gezeigt. Die Ventilationsmaschinen 4 weisen jeweils einen Stator 5 und einen Rotor 6 auf.

Nachfolgend werden Merkmale der Verarbeitungsanlage 1 anhand von Ausführungsbeispielen mit einer unterschiedlichen Anzahl von Ventilationsmaschinen 4 beschrieben. Merkmale der Ventilationsmaschinen 4 werden für eine bessere Übersichtlichkeit insbesondere anhand einer einzelnen Ventilationsmaschine 4 beschrieben. Diese Merkmale können einzeln oder in Kombination auf einige oder alle der übrigen Ventilationsmaschinen einer Verarbeitungsanlage 1 übertragen werden.

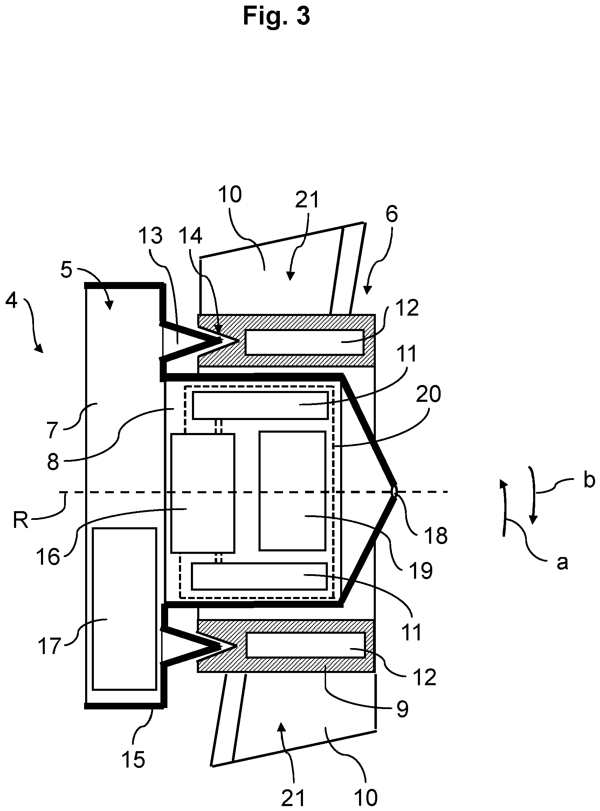

Wie insbesondere in Figur 2 oder Figur 3 zu erkennen ist, ist der Rotor 6 magnetisch berührungslos an dem Stator 5 gelagert.

Figur 2 zeigt eine Verarbeitungsanlage 1 mit lediglich einer Ventilationsmaschine 4, wobei die Ventilationsmaschine 4 in einem Längsschnitt dargestellt ist.

Der Stator 5 der in Figur 2 gezeigten Ventilationsmaschine 4 ist ein separates Bauteil, das mit einer Wandung der Einhausung 3 mechanisch verbunden ist. Der Stator 5 weist einen Fußabschnitt 7 und einen von dem Fußabschnitt 7 in die kontrollierte Umgebung 2 hineinragenden, zylindrisch ausgebildeten Kopfabschnitt 8 auf.

Die Verbindung des Stators 5 mit der Wandung der Einhausung 3 ist derart ausgebildet, dass eine geschlossene Oberfläche an der Grenze des Fußabschnitts 7 des Stators 5 zu der Wandung gebildet ist. Die Verbindung ist frei von Spalten, Fugen und anderen Unebenheiten. Dazu kann der Stator 5 beispielsweise mit einer Schicht, insbesondere einer Kunststoffschicht, überzogen sein, die nahtlos an die Wandung der Einhausung 3 anliegt (in Figur 2 nicht gezeigt).

Der Rotor 6 der in Figur 2 gezeigten Ventilationsmaschine 4 ist ebenfalls ein separates Bauteil, das an dem mit der Wandung der Einhausung 3 verbundenen Stator 5 magnetisch berührungslos gelagert ist. Der Rotor 6 weist einen insbesondere hohlzylindrisch ausgebildeten Ringabschnitt 9 und eine Mehrzahl von an dem Ringabschnitt 9 angeordneten, radial hervorragenden Rotorblättern 10 auf.

Der Rotor 6 ist mit seinem Ringabschnitt 9 auf den Kopfabschnitt 8 des Stators 5 aufgesetzt. Mit anderen Worten ausgedrückt, umgibt der Rotor 6 den Stator in einer Umfangsrichtung. Die hier beschriebene Ventilationsmaschine 4 ist somit in der Art eines als Außenläufer bezeichneten Elektromotors ausgebildet.

Aufgrund der magnetisch berührungslosen Lagerung des Rotors 6 ist ein fluidal durchströmbarer Spalt 7 zwischen dem Stator 5 und dem Rotor 6, insbesondere zwischen dem Kopfabschnitt 8 des Stators 5 und dem Ringabschnitt 9 des Rotors 6 ausgebildet.

Zur Realisierung der magnetischen Lagerung des Rotors 6 weist der Stator 5 mindestens eine als Statorwicklung bezeichnete, insbesondere in Figur 3 zu erkennende Leiterspule 11 auf. Die Leiterspule 11 ist insbesondere in dem Kopfabschnitt 8 angeordnet. Ferner weist der Rotor 6 mindestens ein Element aus einem magnetischen Material 12 auf, das/die in dem Ringabschnitt 9 angeordnet ist/sind und den Kopfabschnitt 8 des Stators 5 ringförmig umgibt/umgeben, wenn der Rotor 6 an dem Stator 5 angeordnet ist. Die Leiterspule 11 und das Element aus einem magnetischen Material 12 wirken derart zusammen, dass ein elektrischer Stromfluss durch die Leiterspule 11 ein Magnetfeld 40 erzeugt, welches das Element aus einem magnetischen Material 12 radial symmetrisch abstößt oder anzieht und somit den Rotor 6 in einer vordefinierten Position an dem Stator 5 in einer Schwebe hält. Zusätzlich kann mit dem Magnetfeld 40 eine Drehbewegung des Rotors 6 um eine Rotationsachse R erzeugt werden. Mit anderen Worten ausgedrückt, existiert keine kontaktierende Verbindung zwischen dem Stator 5 und dem Rotor 6. Daher kann der Rotor 6 ohne Reibungsverluste und verschließfrei rotieren. Es werden keine Gleit- oder Schmiermittel benötigt, die zu einem Eintrag von Verunreinigungen in die kontrollierte Umgebung 2 führen können. Ferner kann der Rotor 6 auf einfache Weise von dem Stator 5 abgenommen werden. Es wird explizit darauf hingewiesen, dass die Darstellung des Magnetfelds 40 in den Figuren nur schematisch ist und dass das Magnetfeld 40 auch anders als gezeigt ausgebildet sein kann.

In Abhängigkeit von einer Steuerung des Stromflusses in der mindestens einen Leiterspule 11 kann der Rotor 6 um die Rotationsachse R in einer ersten Drehrichtung a im Uhrzeigersinn oder in einer zweiten Drehrichtung b entgegen dem Uhrzeigersinn gedreht werden.

Die Rotorblätter 10 des Rotors 6 sind insbesondere derart ausgebildet, dass eine Atmosphäre der kontrollierten Umgebung 2 in Abhängigkeit einer Drehrichtung a oder b des Rotors auf unterschiedlichen Wegen von der Ventilationsmaschine 4 angesaugt und abgeblasen wird.

Bei einer Drehung des Rotors 6 in der ersten Drehrichtung a, die in Figur 2 angedeutet ist, kann die Atmosphäre beispielsweise axial, d.h. parallel zu der Rotationsachse R, angesaugt und zumindest anteilig radial, d.h. transversal zu der Rotationsachse R, von dem Rotor 6 abgeblasen werden, wie anhand der eingezeichneten gestrichelten Pfeile zu erkennen ist. Insbesondere kann die Atmosphäre von einer von dem Fußabschnitt 7 abgewandten Seite des Rotors 6 angesaugt und im Wesentlichen orthogonal zu der Anströmrichtung von dem Rotor 6 abgeblasen werden. Auf diese Weise kann stromabwärts des Rotors 6 eine großvolumige Strömung erzielt werden, mit welcher eine schnelle Umwälzung der Atmosphäre möglich ist.

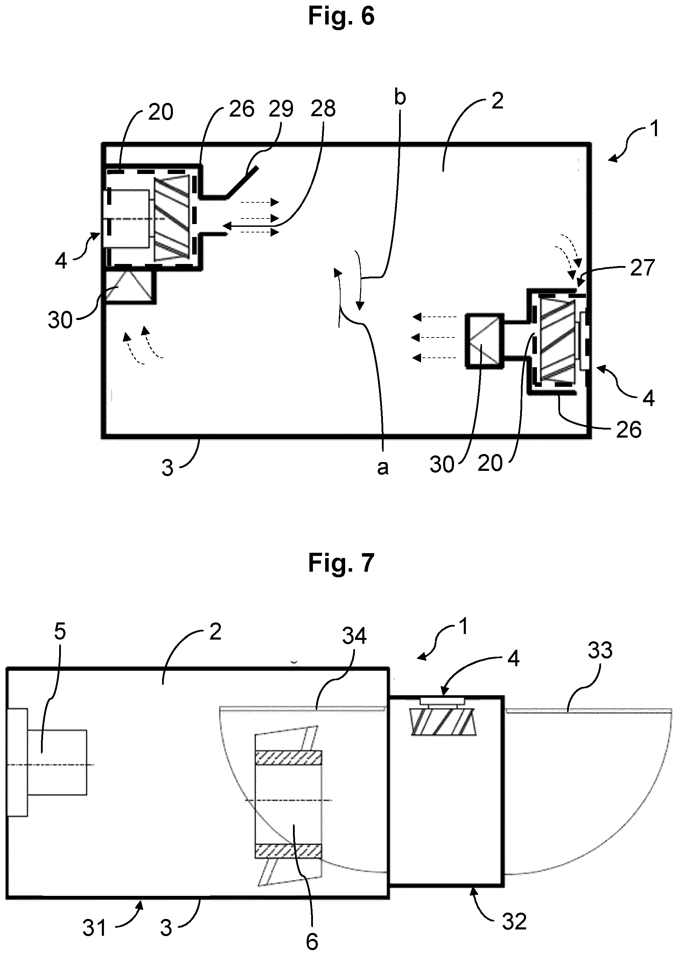

Bei einer Drehung des Rotors 6 in der zweiten Drehrichtung b, die nicht in Figur 2, aber in Figur 6, gezeigt ist, kann die Atmosphäre beispielsweise axial, d.h. parallel zu der Rotationsachse R, angesaugt und zumindest anteilig axial abgeblasen werden. Insbesondere kann die Atmosphäre von einer dem Fußabschnitt 7 zugewandten Seite des Rotors 6 axial angesaugt und auf einer von dem Fußabschnitt 7 abgewandten Seite des Rotors 6 im Wesentlichen parallel zu der Rotationsachse R werden. Auf diese Weise kann stromabwärts des Rotors 6 eine Strömung mit hohem Druck erzielt werden, mit welcher eine Reinigung der kontrollierten Umgebung 2 möglich ist. Insbesondere können mit dieser Strömung Ablagerungen von Verschmutzungen, Partikel wie Staub, Pulver und/oder Flüssigkeiten, und/oder in der Verarbeitungsanlage verarbeitete pharmazeutische Erzeugnisse erfasst, bewegt und insbesondere entfernt werden.

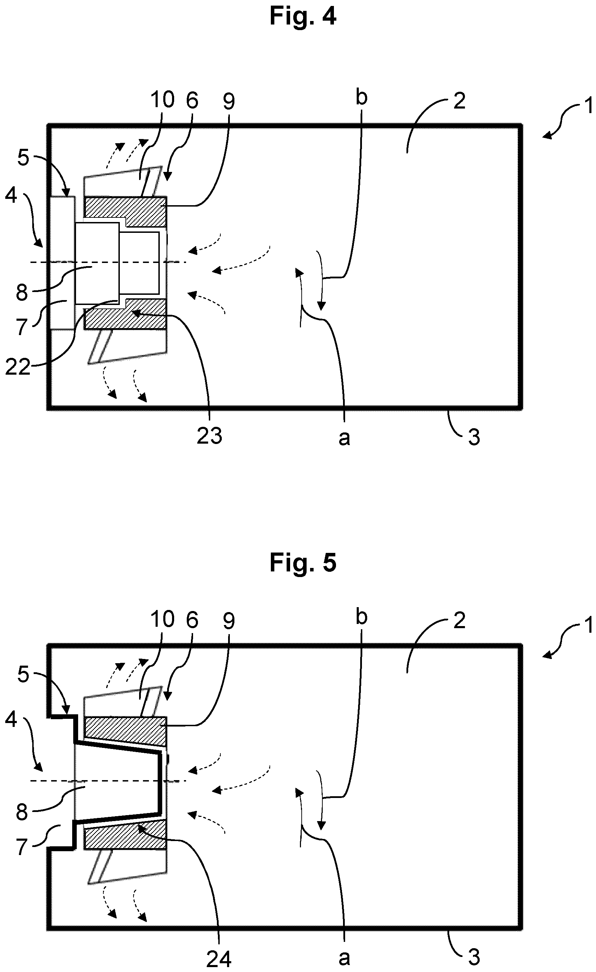

Damit der Rotor 6 auf einfache Weise richtig an dem Stator 5 angeordnet werden kann, weist die Stator-und-Rotor-Kombination eine geometrische Codierung auf. Bei dem in Figur 3 gezeigten Beispiel kann die Codierung mindestens einen an dem Fußabschnitt 7 ausgebildeten und parallel zu der Rotationsachse R in Richtung des Rotors 6 hervorragenden kegelstumpfförmig Abschnitt 13 aufweisen. Der Rotor 6 weist an dem Ringabschnitt 9 auf einer im eingesetzten Zustand des Rotors 6 zu dem Fußabschnitt 7 weisenden Seite eine Gegenform 14 auf, welche den mindestens einen kegelstumpfförmigen Abschnitt 13 aufnehmen kann. Es sind auch andere Ausbildungen der Codierung möglich, wie beispielsweise im Zusammenhang mit den Figuren 4 und 5 im Detail beschrieben.

Der Stator 5 weist eine insbesondere in Figur 3 gezeigte Einkapselung 15 auf. Der Rotor ist vorliegend aus Vollmaterial gebildet. Aufgrund der Einkapselung 15 und der Ausbildung aus Vollmaterial sind die Oberfläche des Stators 5 und des Rotors 6 geschlossenen und glatt. Die Einkapselung 15 ist vorliegend insbesondere in Form einer dicht anliegenden Haut auf dem Stator 6 ausgebildet. Sie kann beispielsweise aus einer aufgetragenen Beschichtung oder aus einem elastischen, aufgezogenen Kunststoff gebildet sein.

Wie insbesondere in Figur 3 zu erkennen ist, weist der Stator 5, beispielsweise in dem Kopfabschnitt 8, einen abstrahiert dargestellten und als Akkumulator ausgebildeten Energiespeicher 16 auf. Mit dem Energiespeicher 16 kann ein der hier beschriebene elektrische Strom in der Leiterspule 11 erzeugt werden.

Der Energiespeicher 16 kann zusätzlich oder alternativ auch für eine Versorgung eines beispielsweise in dem Fußabschnitt 7 des Stators 5 angeordneten Kommunikationsmoduls 17 verwendet werden. Das Kommunikationsmodul 17 kann beispielsweise eine Schnittstelle für den Empfang und das Senden von Daten aufweisen (nicht gezeigt). Zusätzlich kann das Kommunikationsmodul 17 eine Datenverarbeitungseinrichtung (nicht gezeigt) aufweisen. Mit dem Kommunikationsmodul 17 können Befehle zur Steuerung einer Bewegung des Rotors 6 empfangen und verarbeitet werden. Diese Befehle können insbesondere das Starten, das Stoppen, eine Vorgabe der Drehrichtung und/oder eine Vorgabe der Drehgeschwindigkeit des Rotors 6 umfassen.

Die in Figur 3 beschriebene Ausführungsform der Ventilationsmaschine 4 ist Bestandteil einer Reinigungseinrichtung und einer Dekontaminationseinrichtung.

Die Ausbildung der Ventilationsmaschine 4 als Bestandteil der Reinigungseinrichtung kann bereits durch die Möglichkeit, den Rotor 6 in der zweiten Drehrichtung b zu drehen, realisiert sein. Denn insbesondere durch das Drehen des Rotors 6 in der zweiten Drehrichtung b kann die kontrollierte Umgebung 2 gereinigt werden, wie beschrieben.

Die Ventilationsmaschine 4 weist einen als abstrahiert dargestellte Düse ausgebildeten Auslass 18 auf, aus welchem selektiv eine Flüssigkeit auf Wasserbasis oder eine Dekontaminationssubstanz ausgeführt werden kann. Der Auslass 18 ist in dem in Figur 3 gezeigten Ausführungsbeispiel an einer Stirnfläche des Kopfabschnitts 8 des Stators 5 ausgebildet. Der Auslass 18 grenzt insofern an einer mit dem Stator 5 und dem Rotor 6 erzeugten Strömung an. Somit kann über den Auslass 18 die Flüssigkeit auf Wasserbasis und/oder die Dekontaminationssubstanz bedarfsweise unmittelbar in die erzeugte Strömung ausgeführt werden.

Die Flüssigkeit auf Wasserbasis kann beispielsweise im Rahmen der Ausbildung der Ventilationsmaschine 4 als Bestandteil der Reinigungseinrichtung genutzt werden. Wenn der Rotor 6 in der zweiten Drehrichtung b gedreht wird und dadurch die beschriebene Reinigung der kontrollierten Umgebung 2 durchgeführt werden soll, kann, wenn zusätzlich die Flüssigkeit auf Wasserbasis aus dem Auslass 18 in die erzeugte Strömung ausgeführt wird, die erzielte Reinigungsleistung erhöht werden. Der Auslass 18 ist dann ebenfalls Bestandteil der Reinigungseinrichtung.

Zusätzlich oder alternativ kann eine Feuchtigkeit in der Atmosphäre bedarfsweise erhöht werden, wenn aus dem Auslass 18 die Flüssigkeit auf Wasserbasis ausgeführt wird, insbesondere wenn dabei der Rotor 6 gedreht wird. Die Atmosphäre mit erhöhter Feuchtigkeit kann besonders schnell in der kontrollierten Umgebung verteilt werden, wenn die Flüssigkeit mit der Düse zerstäubt wird und/oder wenn der Rotor 6 in der ersten Drehrichtung a gedreht wird, weil durch eine Drehung in der ersten Drehrichtung a eine großvolumige Strömung erzeugt werden kann, wie beschrieben.

Die Dekontaminationssubstanz, beispielsweise Wasserstoffperoxid, kann beispielsweise im Rahmen der Ausbildung der Ventilationsmaschine als Bestandteil der Dekontaminationseinrichtung genutzt werden. Wenn aus dem Auslass 18 beispielsweise die Dekontaminationssubstanz ausgeführt wird oder ausgeführt worden ist und insbesondere wenn der Rotor 6 in der ersten Drehrichtung a gedreht wird, kann die Dekontaminationssubstanz gemeinsam mit der umgewälzten Atmosphäre besonders schnell in der kontrollierten Umgebung 2 verteilt werden. Der Auslass 18 ist dann ebenfalls Bestandteil der Dekontaminationseinrichtung.

Bei einer alternativen, hier nicht gezeigten Ausbildung der Ventilationsmaschine können mehrere Auslässe vorgesehen sein, die an unterschiedlichen Positionen in Bezug auf den Rotor 6 angeordnet sein können und/oder aus denen jeweils ein einziger vorbestimmter Stoff ausführbar ist. Beispielsweise kann ein erster Auslass speziell für die Ausfuhr einer Flüssigkeit auf Wasserbasis realisiert sein und ein zweiter Auslass speziell für die Ausfuhr einer Dekontaminationssubstanz realisiert sein. Alternativ können der erste und der zweite Auslass beide für die Ausfuhr einer Flüssigkeit auf Wasserbasis oder einer Dekontaminationssubstanz realisiert sein. Zusätzlich oder wieder alternativ kann der erste Auslass an der Stirnfläche des Kopfabschnitts 8 des Stators 5 ausgebildet sein und/oder der zweite Auslass in dem Fußabschnitt 7 des Stators 5 oder in der Wandung der Einhausung 3 ausgebildet sein.

Für eine Möglichkeit, die Dekontaminationssubstanz, insbesondere eine flüssige Dekontaminationssubstanz, in besonders kleine Partikel überführen zu können, und/oder die Dekontaminationssubstanz noch schneller in der kontrollierten Umgebung 2 verteilen zu können, weist die Verarbeitungsanlage 1 eine Vibrationseinrichtung 19 zum Vaporisieren der Dekontaminationssubstanz auf. Die Vibrationseinrichtung 19 ist als eine Ultraschalleinrichtung in dem Stator 5 ausgebildet. Sie umfasst ein Vibrationselement (nicht gezeigt), das in dem Stator 5 angeordnet ist, und eine an dem Vibrationselement anliegende Vibrationsplatte (nicht gezeigt), die einen Teil der Oberfläche des Stators 5 ausbildet und in fluidalem Kontakt mit einer von der Ventilationsmaschine 4 erzeugten Strömung steht. Das Vibrationselement kann insbesondere im Ultraschall-Frequenzbereich vibrieren und eine Schwingung der Vibrationsplatte im Ultraschall-Frequenzbereich anregen. Die Vibrationsplatte kann somit eine Ultraschallplatte sein.

Bei einem Kontakt der flüssigen Dekontaminationssubstanz mit der im Ultraschall-Frequenzbereich vibrierenden Vibrationseinrichtung 19 wird die Dekontaminationssubstanz aufgrund der hochfrequenten Vibration vaporisiert oder zunächst in kleine Tröpfchen, insbesondere in ein Aerosol, überführt und anschließend verdampft. So kann die Dekontaminationssubstanz besonders gleichmäßig, gezielt und/oder schnell in der kontrollierten Umgebung 2 verteilt werden.

Die Verarbeitungsanlage 1 kann zusätzlich eine Vorrichtung zum Ionisieren der vorzugsweise vaporisierten Dekontaminationssubstanz aufweisen (nicht dargestellt).

Die Ventilationsmaschine 4 weist zudem eine als Widerstandsheizung ausgebildete Beheizungseinrichtung 20 auf. Die Widerstandsheizung ist als ein mit dem Energiespeicher 16 elektrisch leitend verbundener elektrischer Leiter ausgebildet, wie in Figur 3 anhand der gepunkteten Linie gezeigt ist. Der elektrische Leiter ist unterhalb der Oberfläche des Stators 5 entlanggeführt. Aufgrund einer Erwärmung des elektrischen Leiters infolge dessen elektrischen Widerstands wird die Oberfläche des Stators 5 erwärmt. Diese Wärme wird auf die zumindest abschnittsweise an dem Stator 5 vorbeiströmende Atmosphäre übertragen. Alternativ oder zusätzlich ist es auch möglich, dass die Leiterspule 11 und/oder das Element aus einem magnetischen Material 12 die Beheizungseinrichtung 20 ist.

Mit der Beheizungseinrichtung 20 ist es möglich, eine vordefinierte Temperatur in der kontrollierten Umgebung 2 und insbesondere in der Atmosphäre einzustellen. Insbesondere kann durch eine mit der Beheizungseinrichtung 20 eingestellten Temperatur auch eine Feuchtigkeit in der kontrollierten Umgebung vordefiniert beeinflusst werden. Wenn die Beheizungseinrichtung 20 betrieben wird und eine Flüssigkeit auf Wasserbasis aus dem Auslass 18 ausgeführt wird, kann die Feuchtigkeit in der erwärmten kontrollierten Umgebung 2 steigen. Wenn die Beheizungseinrichtung 20 betrieben wird, ohne dass eine Flüssigkeit auf Wasserbasis 17 aus dem Auslass 18 ausgeführt wird, kann die kontrollierte Umgebung 2 getrocknet werden und somit die Feuchtigkeit in der kontrollierten Umgebung 2 sinken.

Wie beschrieben, weist der Stator 5 der in Figur 3 gezeigten Ausführungsform die Einkapselung 15 und der Rotor 6 die Ausbildung aus einem Vollmaterial auf, so dass die Oberflächen des Stators 5 und des Rotors 6 geschlossenen und glatt sind. Dadurch stehen in vorteilhafter Weise nur wenige Möglichkeiten zur Ablagerung von Verschmutzungen, Resten der Dekontaminationssubstanz und/oder ähnlichen unerwünschten Ablagerungen zur Verfügung.

Die Ventilationsmaschine kann zusätzlich, insbesondere auf der Oberfläche des Rotors 6, eine katalytische Beschichtung 19 aus einem katalytischen Material, nämlich aus Manganoxid, aufweisen, wie beispielsweise in Figur 3 gezeigt. Die katalytische Beschichtung 19 steht mit einer mit dem Rotor 6 erzeugten Strömung fluidal in Kontakt.

Wenn die umgewälzte Atmosphäre und die in der Atmosphäre enthaltene Dekontaminationssubstanz mit der katalytischen Beschichtung 19 in Kontakt gelangen, kann eine Aufspaltung der als Wasserstoffperoxid ausgebildeten Dekontaminationssubstanz erfolgen. Diese Aufspaltung kann insbesondere für ein Entfernen zumindest eines Anteils der Dekontaminationssubstanz aus der kontrollieren Umgebung 2 zielführend sein. Aufgrund der einfachen Austauschbarkeit des Rotors 6 der Ventilationseinrichtung 4 kann beispielsweise für ein Verteilen der Dekontaminationssubstanz in der kontrollierten Umgebung 2 ein Rotor 6 ohne die katalytische Beschichtung verwendet werden. Wenn die Dekontamination der kontrollierten Umgebung 2 abgeschlossen ist, kann der Rotor 6 ohne die katalytische Beschichtung 19 entnommen und ein Rotor 6 mit der katalytischen Beschichtung 19 auf den Stator 5 aufgesetzt und gestartet werden.

In Figur 4 und Figur 5 sind alternative Ausführungen der Ventilationsmaschine gezeigt. Diese alternativen Ausführungsformen unterscheiden sich von der in Figur 2 gezeigten Ausführungsform insbesondere durch die Ausbildung der beschriebenen geometrischen Codierung.

Bei der in Figur 4 gezeigten Stator-und-Rotor-Kombination weist der Stator 5 in einem Übergangsbereich von dem Fußabschnitt 7 zu dem Kopfabschnitt 8 einen gestuften Absatz 22 auf. Der Rotor 6 weist an dem Ringabschnitt 9 auf einer im eingesetzten Zustand des Rotors 6 zu dem Kopfabschnitt 8 weisenden Seite eine Gegenform 23 auf, welche den gestuften Absatz 22 aufnehmen kann.

Bei der in Figur 5 gezeigten Stator-und-Rotor-Kombination ist der Kopfabschnitt 8 des Stators 5 kegelstumpfförmig ausgebildet. Der Ringabschnitt 9 des Rotors weist auf einer in Richtung der Rotationsachse R weisenden Seite eine Gegenform 24 auf, welche den kegelstumpfförmigen Kopfabschnitt 8 in ausschließlich einer Orientierung des Rotors 6 aufnehmen kann.

Die in Figur 5 gezeigte Ausführungsform der in der Verarbeitungsanlage 1 vorgesehen Ventilationsmaschine 4 unterscheidet sich ferner von den in den Figuren 1 bis 4 gezeigten Ausführungsformen, dadurch dass die in Figur 5 gezeigte Ausführungsform einen Stator 5 aufweist, der als ein in die kontrollierte Umgebung 2 hineinragender Bestandteil der Einhausung 3 ausgebildet ist. Der Stator 5 ist folglich nicht als separates Bauteil ausgebildet, sondern einstückig mit der Wandung der Einhausung 3 ausgebildet. Die Wandung der Einhausung 3 umfasst eine Ausstülpung 25 in Gestalt des separat ausgebildeten Stators 5 oder des Kopfabschnitts 8 des separat ausgebildeten Stators, in welcher die hier beschriebenen, in dem separat ausgebildeten Stator 5 angeordneten Bauteile integriert sind.

In Figur 6 ist eine weitere Ausführungsform der Verarbeitungsanlage 1 gezeigt. Die in Figur 6 gezeigte Verarbeitungsanlage 1 weist eine kontrollierte Umgebung 2 und eine die kontrollierte Umgebung 2 umgebende Einhausung 3 auf. In der kontrollierten Umgebung 2 sind zwei Ventilationsmaschinen 4 angeordnet, die beispielsweise einige oder alle der in Zusammenhang mit den Figuren 1 bis 5 beschrieben Merkmale aufweisen können. Die Rotoren 6 der beiden in Figur 6 gezeigten Ventilationsmaschinen 4 werden in der zweiten Drehrichtung b gedreht, so dass abströmseitig eine axiale Strömung damit erzeugt wird, wie anhand der eingezeichneten gestrichelten Pfeile zu erkennen ist.

Zusätzlich sind die in Figur 6 gezeigten Ventilationsmaschinen 4 jeweils in einem Gehäuse 26 angeordnet. Das Gehäuse 26 umgibt die darin angeordnete Ventilationsmaschine 4 und schützt diese vor einer mechanischen Beschädigung. Jedes der Gehäuse 26 besitzt zumindest eine Zuströmöffnung 27 und eine Ausströmöffnung 28, damit die Atmosphäre der kontrollieren Umgebung 2 mit der Ventilationsmaschine 4 umgewälzt werden kann.

Das Gehäuse kann zumindest abschnittsweise als Leiterfläche zum fluidalen Leiten der durch das Gehäuse 26 strömenden Atmosphäre dienen.

Zusätzlich kann an der Zuströmöffnung 27 und/oder an der Ausströmöffnung 28 ein Verschlussstück 29 angeordnet sein. Bei der in Figur 6 gezeigten Ausführungsform der Verarbeitungsanlage 1 weist nur das auf der linken Seite gezeigte Gehäuse 26 ein Verschlussstück 29 an der Ausströmöffnung 28 auf. Das Verschlussstück 29 kann insbesondere als eine schwenkbare Platte ausgebildet sein, die eine Zuströmöffnung 27 oder eine Ausströmöffnung 28, an der die Platte angrenzt, freigeben und verschließen kann. Das Verschlussstück 29 kann, insbesondere wenn es als schwenkbare Platte an der Ausströmöffnung 28 ausgebildet ist, auch als Leiterfläche zum fluidalen Leiten der durch das Gehäuse 26 strömenden Atmosphäre dienen.

Weiter zusätzlich kann an der Zuströmöffnung 27 und/oder an der Ausströmöffnung 28 eine Filtervorrichtung 30 zum Filtern von Partikeln angeordnet sein. Bei der in Figur 6 gezeigten Ausführungsform der Verarbeitungsanlage 1 ist bei dem auf der linken Seite gezeigten Gehäuse 26 eine Filtervorrichtung 30 an der Zuströmöffnung 27 angeordnet und bei dem auf der rechten Seite gezeigten Gehäuse 26 eine Filtervorrichtung 30 an der Ausströmöffnung 28 angeordnet. Die Filtervorrichtungen 30 tragen dazu bei, dass die kontrollierte Umgebung 2 eine Reinheit entsprechend der Reinraumklasse A oder eine Reinheit gemäß einer der Reinraumklassen 1 bis 5 gemäß DIN EN ISO 14644-1 aufweisen kann.

Die der in Figur 6 gezeigten Ausführungsform der Verarbeitungsanlage 1 ist eine Beheizungsvorrichtung 31 an der Innenwandung jedes der eine Ventilationsmaschine 4 umgebenden Gehäuses 26 angeordnet. Diese Beheizungseinrichtung 31 kann analog zu der in dem Stator 5 angeordneten Beheizungseinrichtung 20 ausgebildet sein.

Wie in Figur 7 gezeigt ist, kann die Einhausung 3 der Verarbeitungsanlage 1 eine Hauptkammer 31 und eine Nebenkammer 32 aufweisen und insbesondere umgeben. Die Hauptkammer 31 ist eine Kammer der Verarbeitungsanlage 1, in welcher eine Verarbeitung einer Substanz, eines Materials, einer Probe oder ähnlichem erfolgt. Die Nebenkammer 32 ist in der in Figur 7 gezeigten Ausführungsform der Verarbeitungsanlage 1 als eine Schleusenkammer 32 mit zwei hintereinander angeordneten und separat öffenbaren Türen 33, 34 ausgebildet. Die erste Tür 33 ist dazu ausgebildet und angeordnet, eine Öffnung in der Einhausung 3 abzudecken oder freizugeben, die die Schleusenkammer 32 fluidal mit einem die Verarbeitungsanlage 1 umgebenden Raum verbindet. Die zweite Tür 34 ist dazu ausgebildet und angeordnet, eine Öffnung in der Einhausung 3 abzudecken oder freizugeben, die die Schleusenkammer 32 fluidal mit der Hauptkammer 31 verbindet. Zumindest die zweite Tür 33, vorzugsweise auch die erste Tür 32, sind druckdichte Türen. Wenn die druckdichte zweite Tür 33 oder die druckdichten Türen 32, 33 geschlossen sind, ist die kontrollierte Umgebung 2 in der Hauptkammer 31 unabhängig von einem Druck in einem die Verarbeitungsanlage umgebenden Raum.

Die Ventilationsmaschine 4, der Rotor 6 und/oder oder eine in der Hauptkammer 31 verarbeitete Substanz, Material und/oder Probe können durch die Schleusenkammer 32 in die Hauptkammer 31 eingebracht werden. Insbesondere wird zu diesem Zweck zunächst die erste Tür 32 geöffnet und die vorgenannten Gegenstände in die Schleusenkammer 32 eingebracht. Anschließend wird die erste Tür 32 geschlossen und daraufhin eine Atmosphäre in der Schleusenkammer 32 gereinigt. Erst danach wird die zweite Tür 33 geöffnet und die vorgenannten Gegenstände von der Schleusenkammer 32 in die Hauptkammer 31 eingebracht. Das Einbringen kann händisch, mit einem bewegbaren Roboter und/oder mit einem magnetischen Fördersystem erfolgen (in Figur 7 nicht gezeigt). Mit dem Einbringen von Gegenständen durch die Schleusenkammer 32 in die Hauptkammer 31 auf die beschriebene Weise ist es möglich, dass ein unerwünschter Eintrag von Partikeln, Teilchen und/oder Substanzen in die Hauptkammer reduziert oder verhindert wird.

Zusätzlich oder alternativ zu der Möglichkeit, die Ventilationsmaschine 4 und/oder den Rotor 6 durch die Schleusenkammer 32 in die Hauptkammer 31 einzubringen, kann eine Ventilationsmaschine 4 in der Schleusenkammer 32 eingerichtet sein, wie in Figur 7 gezeigt ist. Die in der Nebenkammer 32 angeordnete Ventilationsmaschine 4 kann beispielsweise einige oder alle der in Zusammenhang mit den Figuren 1 bis 6 beschrieben Merkmale aufweisen. Mit der in der Nebenkammer 32 angeordneten Ventilationsmaschine 4 können die in Verbindung mit der Ventilationsmaschine 4 beschriebenen Wirkungen zusätzlich in der Nebenkammer 32 erzielt werden.

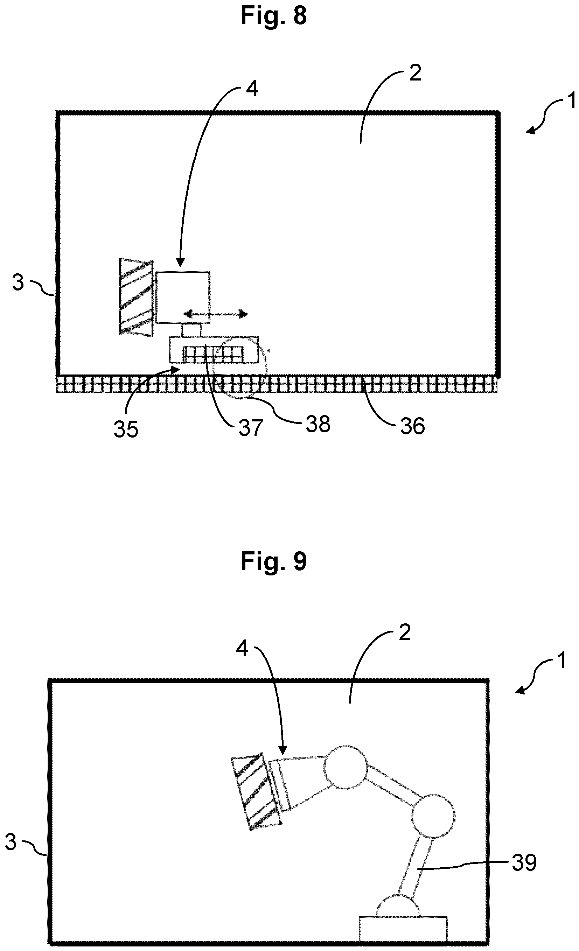

Wie insbesondere in Figur 8 und Figur 9 gezeigt ist, kann die Ventilationsmaschine 4 ortsveränderlich angeordnet sein. Der Stator 5 ist dann nicht mechanisch starr mit der Wandung der Einhausung 3 verbunden, sondern mit einem bewegbaren Element.

Bei der in Figur 8 gezeigten Ausführungsform der Verarbeitungsanlage 1 ist die Ventilationsmaschine 4 auf einem Magnetmover 37 eines magnetischen Fördersystems 35 angeordnet. Das magnetische Fördersystem 35 umfasst einen modular ausgebildeten Magnetfelderzeuger 36, der insbesondere in Form eines Planarmotors ausgebildet sein kann, und den mindestens einen Magnetmover 37, der auf dem Magnetfelderzeuger 36 transportiert werden kann. Der Magnetmover 37 levitiert dabei über dem Magnetfelderzeuger 36. Die Ventilationsmaschine 4 ist auf dem Magnetmover 37 angeordnet und kann mit dem Magnetmover 37 ortsveränderlich bewegt werden.

Der Antrieb des Magnetmovers 37 wird insbesondere durch eine magnetische Wechselwirkung zwischen dem Magnetfelderzeuger 36 und der Magnetmover 37 erzeugt. Insbesondere wird ein Magnetfeld 38 zwischen dem Magnetmover 37 und dem Magnetfelderzeuger 36 erzeugt, mit welchem der Magnetmover 37 in der Schwebe über dem Magnetfelderzeuger 36 gehalten und schwebend über dem Magnetfelderzeuger 36 geführt werden kann. Der Magnetmover 37 dienst somit in der Art eines Schlittens. Es wird explizit darauf hingewiesen, dass die Darstellung des Magnetfelds 38 in den Figuren nur schematisch ist und dass das Magnetfeld 38 auch anders als gezeigt ausgebildet sein kann.

Mit der Anordnung der Ventilationsmaschine 4 auf dem magnetischen Fördersystem 35 kann die Ventilationsmaschine 4 besonders flexibel insbesondere in der kontrollierten Umgebung 2 transportiert werden.

Bei der in Figur 9 gezeigten Ausführungsform der Verarbeitungsanlage 1 ist die Ventilationsmaschine 4 an einem bewegbaren Roboterarm 39 angeordnet. Der Roboterarm 39 kann mit einem ersten Ende an der Einhausung 3 fixiert sein. Ein zweites Ende des Roboterarms 39, an welchem die Ventilationsmaschine 3 angeordnet ist, kann in allen drei Raumrichtungen in der kontrollierten Umgebung 2 bewegt werden. Mit der Anordnung der Ventilationsmaschine 4 an dem Roboterarm 39 kann die Ventilationsmaschine 4 besonders flexibel bewegt werden.

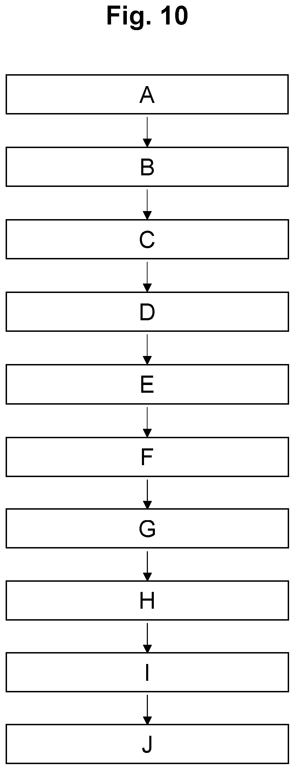

In Figur 10 ist zusätzlich zu der Verarbeitungsanalage mit den hier beschriebenen, einzeln oder in Kombination realisierbaren Merkmalen ein Verfahren zum Behandeln einer kontrollierten Umgebung der Verarbeitungsanalage in einer möglichen Ausführungsform beschrieben.

Das hier beschriebene Verfahren ist nur eine von mehreren Möglichkeiten zum Behandeln einer kontrollierten Umgebung der Verarbeitungsanalage. Für mögliche Abwandlungen von dem hier beschriebenen Verfahren wird explizit auch auf die im Zusammenhang mit der Verarbeitungsanlage beschriebenen Vorrichtungsmerkmale und die damit verbundenen Verfahrensschritte und technischen Effekte verwiesen.

Das Verfahren zum Behandeln einer kontrollierten Umgebung einer Verarbeitungsanlage sieht zunächst ein Einrichten der Verarbeitungsanlage und insbesondere der Ventilationsmaschine vor.

Zu diesem Zweck wird in einem ersten Schritt A die Ventilationsmaschine oder zumindest der Rotor der Ventilationsmaschine händisch durch einen Handschuheingriff der Verarbeitungsanlage, welcher in einer Schleusenkammer angeordnet ist, und insbesondere durch eine Öffnung in einer Einhausung der Verarbeitungsanlage in die kontrollierte Umgebung eingeführt. Die Ventilationsmaschine oder zumindest der Rotor kann dabei bestimmungsgemäß positioniert werden. Beispielsweise kann die Ventilationsmaschine auf einem magnetischen Fördersystem, einem Roboterarm oder an einer Wandung der Einhausung angeordnet werden. Wenn nur der Rotor durch die Öffnung in die kontrollierte Umgebung eingeführt wird, kann der Rotor an dem insbesondere bereits in der kontrollierten Umgebung befindlichen Stator angeordnet werden.

In einem zweiten Verfahrensschritt B ruht die Ventilationsmaschine nach der Einnahme der bestimmungsgemäßen Position zunächst für eine vorbestimmte Dauer.

In einem dritten Verfahrensschritt C wird eine Flüssigkeit auf Wasserbasis, insbesondere eine ein Detergens in Form von Seifenlauge enthaltende wässrige Lösung, durch einen in der kontrollierten Umgebung angeordneten Auslass in die kontrollierte Umgebung eingebracht.

In einem vierten Verfahrensschritt D wird der Rotor gedreht und die Flüssigkeit auf Wasserbasis mittels der Ventilationsmaschine in der kontrollierten Umgebung verteilt. Dabei kann insbesondere eine turbulente, axial zur Drehachse des Rotors ausgerichtete Strömung mit der Ventilationsmaschine erzeugt werden. Mittels der Ventilationsmaschine wird folglich eine Atmosphäre in der kontrollierten Umgebung bewegt. Durch die erzeugte Strömung und unter Mitwirkung der verteilten Flüssigkeit wird die kontrollierte Umgebung mittels der Ventilationsmaschine gereinigt. Wenn die Reinigung abgeschlossen ist, wird der Auslass der Flüssigkeit auf Wasserbasis wieder gestoppt.

In einem fünften Verfahrensschritt E wird mittels der Ventilationsmaschine eine vorbestimmte, insbesondere niedrige, Feuchtigkeit in der kontrollierten Umgebung erzeugt. Dazu wird eine Beheizungseinrichtung aktiviert und die Atmosphäre in der kontrollierten Umgebung erwärmt. Optional kann gleichzeitig der Rotor gedreht werden. Dadurch wird die Atmosphäre getrocknet und eine Feuchtigkeit der Atomsphäre kann auf ca. 25 % oder weniger reduziert werden. Es ist möglich, die Drehrichtung des Rotors wiederkehrend zu wechseln. Der Rotor ist dabei in vorteilhafter Weise so ausgebildet, dass unterschiedliche Drehrichtungen a, b unterschiedliche Strömungen erzeugen, beispielsweise die beschriebene radiale Strömung orthogonal zu der Rotationsachse bei einer Drehung in einer ersten Drehrichtung a und eine axiale Strömung in einer zweiten Drehrichtung b. Durch den wiederkehrenden Wechsel der Drehrichtung kann die Atmosphäre besonders schnell umgewälzt, und somit insbesondere schnell getrocknet, werden.

In einem sechsten Verfahrensschritt F kann die Ventilationsmaschine erneut für eine vorbestimmte Dauer ruhen. Dadurch können die Atmosphäre und in der kontrollierten Umgebung angeordneten Gegenstände ebenfalls zur Ruhe kommen.

In einem siebten Verfahrensschritt G wird eine Dekontaminationssubstanz, insbesondere Wasserstoffperoxid, in flüssiger Form durch den beschriebenen Auslass oder einen anderen Auslass in die kontrollierte Umgebung, insbesondere auf eine Ultraschallplatte einer Vibrationseinrichtung, eingebracht. Die flüssige Dekontaminationssubstanz wird mittels einer Ultraschallbehandlung an der Ultraschallplatte vaporisiert und kann somit schnell und homogen in der kontrollierten Umgebung verteilt werden.

In einem achten Verfahrensschritt H wird die vaporisierte Dekontaminationssubstanz mit der Ventilationsmaschine in der kontrollierten Umgebung verteilt und somit die kontrollierte Umgebung dekontaminiert. Dabei kann insbesondere wieder die Drehrichtung des Rotors wiederkehrend gewechselt werden, und somit der beschriebene, damit verbundenen Vorteil einer schnellen Umwälzung der Atmosphäre ausgenutzt werden. Mittels der Ventilationsmaschine wird folglich auch in diesem Verfahrensschritt eine Atmosphäre in der kontrollierten Umgebung bewegt. Nach dem Abschluss der Dekontamination wird die Ventilationsmaschine gestoppt.

In einem neunten Verfahrensschritt I kann der Rotor der Ventilationsmaschine gegen einen alternativen Rotor ausgetauscht werden. Der alternative Rotor kann insbesondere geometrisch so gestaltet sein, wie der zu Beginn des Verfahrens verwendete Rotor. Allerdings kann der alternative Rotor zusätzlich eine katalytische Beschichtung, beispielsweise Manganoxid, auf der Oberfläche aufweisen.

In einem zehnten Verfahrensschritt J wird in der kontrollierten Umgebung eine reine Atmosphäre, die keine Dekontaminationssubstanz enthält, mit der Ventilationsmaschine verteilt. Die auf der Oberfläche des alternativen Rotors befindliche katalytische Beschichtung trägt dazu bei, dass die Dekontaminationssubstanz aufgespalten wird und somit unschädlich wird. Zusätzlich kann eine neue, frische Atmosphäre durch einen weiteren, insbesondere mit einem Luftfilter versehenen Auslass in die kontrollierte Umgebung eingeblasen und mit der Ventilationsmaschine verteilt werden, um die kontrollierte Umgebung zu spülen.

Danach ist die Verarbeitungsanlage für eine Verarbeitung einer Substanz, eines Materials oder eines Stoffes in der kontrollierten Umgebung eingerichtet.

### Bezugszeichenliste

- 1: Verarbeitungsanlage
- 2: kontrollierte Umgebung
- 3: Einhausung
- 4: Ventilationsmaschine
- 5: Stator
- 6: Rotor
- 7: Fußabschnitt
- 8: Kopfabschnitt
- 9: Ringabschnitt
- 10: Rotorblatt
- 11: Leiterspule
- 12: Element aus einem magnetischen Material
- 13: kegelstumpfförmiger Abschnitt
- 14: Gegenform
- 15: Einkapselung
- 16: Energiespeicher
- 17: Kommunikationsmodul
- 18: Auslass
- 19: Vibrationseinrichtung
- 20: Beheizungseinrichtung
- 21: katalytische Beschichtung
- 22: gestufter Absatz
- 23: Gegenform
- 24: Gegenform
- 25: Ausstülpung
- 26: Gehäuse
- 27: Zuströmöffnung
- 28: Ausströmöffnung
- 29: Verschlussstück
- 30: Filtervorrichtung
- 31: Hauptkammer
- 32: Nebenkammer, Schleusenkammer
- 33: Tür
- 34: Tür
- 35: magnetisches Fördersystem
- 36: Magnetfelderzeuger
- 37: Magnetmover
- 38: Magnetfeld
- 39: Roboterarm
- 40: Magnetfeld

- R: Rotationsachse
- a: Drehrichtung
- b: Drehrichtung

- A: Schritt
- B: Schritt
- C: Schritt
- D: Schritt
- E: Schritt
- F: Schritt
- G: Schritt
- H: Schritt
- I: Schritt
- J: Schritt

## Patentansprüche

1. Verarbeitungsanlage (1) mit einer kontrollierten Umgebung (2) und einer die kontrollierte Umgebung (2) umgebenden Einhausung (3), **dadurch gekennzeichnet, dass** die Verarbeitungsanlage (1) mindestens eine mit der kontrollierten Umgebung (2) fluidal verbundene Ventilationsmaschine (4) mit einem Stator (5) und einem magnetisch berührungslos gelagerten Rotor (6) aufweist.

2. Verarbeitungsanlage (1) dem vorhergehenden Anspruch, **dadurch gekennzeichnet, dass** die Ventilationsmaschine (4) ein Bestandteil einer Dekontaminationseinrichtung ist.

3. Verarbeitungsanlage (1) nach einem der vorhergehenden Ansprüche, **dadurch gekennzeichnet, dass** die Ventilationsmaschine (4) einen Auslass (18), insbesondere eine Einstoffdüse oder eine Zweistoffdüse, aufweist, aus welchem eine Dekontaminationssubstanz, insbesondere Wasserstoffperoxid, in eine mit dem Stator (5) und/oder dem Rotor (6) erzeugte Strömung ausführbar ist.

4. Verarbeitungsanlage (1) nach einem der vorhergehenden Ansprüche, **dadurch gekennzeichnet, dass** der Stator (5) und/oder der Rotor (6) eine geschlossene Oberfläche aufweist/aufweisen.

5. Verarbeitungsanlage (1) nach einem der vorhergehenden Ansprüche, **dadurch gekennzeichnet, dass** die Verarbeitungsanlage eine Vibrationseinrichtung (19) zum Vaporisieren einer Dekontaminationssubstanz, insbesondere eine Ultraschalleinrichtung, aufweist.

6. Verarbeitungsanlage (1) nach einem der vorhergehenden Ansprüche, **dadurch gekennzeichnet, dass** die kontrollierte Umgebung (2) von der Einhausung (3) druckdicht umschließbar ist.

7. Verarbeitungsanlage (1) nach einem der vorhergehenden Ansprüche, **dadurch gekennzeichnet, dass** die kontrollierte Umgebung (2) eine Reinheit gemäß der Reinraumklasse A aufweist.

8. Verarbeitungsanlage (1) nach einem der vorhergehenden Ansprüche, **dadurch gekennzeichnet, dass** die Ventilationsmaschine (4) ein Bestandteil einer Reinigungseinrichtung ist.

9. Verarbeitungsanlage (1) nach einem der vorhergehenden Ansprüche, **dadurch gekennzeichnet, dass** die Ventilationsmaschine (4) derart ausgebildet und angeordnet ist, dass eine turbulente Strömung in der kontrollierten Umgebung (2) erzeugbar ist.

10. Verarbeitungsanlage (1) nach einem der vorhergehenden Ansprüche, **dadurch gekennzeichnet, dass** Leitflächen zur Führung einer erzeugten Strömung ausgebildet sind.

11. Verarbeitungsanlage (1) nach einem der vorhergehenden Ansprüche, **dadurch gekennzeichnet, dass** die Verarbeitungsanlage eine Filtervorrichtung (30) zum Filtern von Partikeln aufweist.

12. Verarbeitungsanlage (1) nach dem vorhergehenden Anspruch, **dadurch gekennzeichnet, dass** der Stator (5) und/oder der Rotor (6) derart ausgebildet ist/sind, dass eine Strömung zumindest anteilig, insbesondere vollständig, radial von der Ventilationsmaschine (4) abgeblasen wird.

13. Verarbeitungsanlage (1) nach einem der vorhergehenden Ansprüche, **dadurch gekennzeichnet, dass** der Stator (5) und/oder der Rotor (6) derart ausgebildet ist/sind, dass eine Strömung zumindest anteilig, insbesondere vollständig, axial von der Ventilationsmaschine (4) abgeblasen wird.

14. Verarbeitungsanlage (1) nach einem der vorhergehenden Ansprüche, **dadurch gekennzeichnet, dass** der Rotor (6) derart ausgebildet ist, dass mit einer Drehung in einer ersten Drehrichtung (a) eine zumindest anteilig radial von der Ventilationsmaschine (4) abgeblasene Strömung erzeugbar ist und dass mit einer Drehung in einer zweiten Drehrichtung (b) eine zumindest anteilig axial von der Ventilationsmaschine (4) abgeblasene Strömung erzeugbar ist.

15. Verarbeitungsanlage (1) nach einem der vorgehenden Ansprüche, **dadurch gekennzeichnet, dass** der Stator (5) und der Rotor (6) derart ausgebildet sind, dass diese beiden Bauteile in einer einzigen Orientierung zueinander aneinander anordbar sind.

16. Verarbeitungsanlage (1) nach einem der vorhergehenden Ansprüche, **dadurch gekennzeichnet, dass** die Stator-und-Rotor-Kombination eine geometrische Codierung hat, insbesondere durch einen kegelstumpfförmig ausgebildeten Abschnitt (8, 13) und/oder einen gestuften Absatz (22) und eine dazu komplementäre Gegenform (14, 23, 24), insbesondere wobei der Rotor (6) in einem Mittelteil die komplementäre Gegenform (14, 23, 24) aufweist.

17. Verarbeitungsanlage (1) nach einem der vorhergehenden Ansprüche, **dadurch gekennzeichnet, dass** die Verarbeitungsanlage, insbesondere die Ventilationsmaschine (4), eine Beheizungseinrichtung (20) aufweist.

18. Verarbeitungsanlage (1) nach einem der vorhergehenden Ansprüche, **dadurch gekennzeichnet, dass** der Stator (5) als ein in die kontrollierte Umgebung (2) hineinragender Bestandteil der Einhausung (3) ausgebildet ist.

19. Verarbeitungsanlage (1) nach einem der vorhergehenden Ansprüche, **dadurch gekennzeichnet, dass** die Ventilationsmaschine (4) ein Gehäuse (26) aufweist.

20. Verarbeitungsanlage (1) nach einem der vorhergehenden Ansprüche, **dadurch gekennzeichnet, dass** die Einhausung (3) eine Hauptkammer (31) und mindestens eine fluidal abtrennbare Nebenkammer (32) aufweist, insbesondere wobei die Nebenkammer (32) eine Zuluftleitung, eine Abluftleitung und/oder eine Schleusenkammer ist, wobei die Ventilationsmaschine (4) in der Nebenkammer (32) angeordnet ist.

21. Verarbeitungsanlage (1) nach einem der vorhergehenden Ansprüche, **dadurch gekennzeichnet, dass** die Ventilationsmaschine (4) ortsveränderlich angeordnet ist.

22. Verarbeitungsanlage (1) nach einem der vorhergehenden Ansprüche, **dadurch gekennzeichnet, dass** die Ventilationsmaschine (4) auf einem auf einem magnetischen Fördersystem (35), insbesondere einem Magnetmover eines magnetischen Fördersystems (35), angeordnet ist.

23. Verarbeitungsanlage (1) nach einem der vorhergehenden Ansprüche, **dadurch gekennzeichnet, dass** die Ventilationsmaschine (4) an einem bewegbaren Roboterarm (38) angeordnet ist.

24. Verarbeitungsanlage (1) nach einem der vorhergehenden Ansprüche, **dadurch gekennzeichnet, dass** die Verarbeitungsanlage, insbesondere die Ventilationsmaschine (4), einen Energiespeicher (16) zur Versorgung der Ventilationsmaschine (4) mit elektrischer Energie aufweist.

25. Verarbeitungsanlage (1) nach einem der vorhergehenden Ansprüche, **dadurch gekennzeichnet, dass** der Stator (5), der Rotor (6) und/oder der Energiespeicher (16) eingekapselt ausgebildet ist/sind.

26. Verarbeitungsanlage (1) nach einem der vorhergehenden Ansprüche, **dadurch gekennzeichnet, dass** mindestens ein Bereich der Verarbeitungsanlage (1), insbesondere mindestens ein Bereich der Ventilationsmaschine (4), eine katalytische Beschichtung (21) aufweist.

27. Verarbeitungsanlage (1) nach einem der vorhergehenden Ansprüche, **dadurch gekennzeichnet, dass** die katalytische Beschichtung (21) ein Manganoxid aufweist oder ist.

28. Verfahren zum Behandeln einer kontrollierten Umgebung (2) einer Verarbeitungsanlage (1) nach einem der vorhergehenden Ansprüche, **dadurch gekennzeichnet, dass** mittels der Ventilationsmaschine (4) eine Atmosphäre in der kontrollierten Umgebung (2) bewegt wird.

29. Verfahren nach dem vorhergehenden Anspruch, **dadurch gekennzeichnet, dass** die Ventilationsmaschine (4) oder zumindest der Rotor (6) der Ventilationsmaschine (4) händisch durch eine Öffnung in einer Einhausung (3) der Verarbeitungsanlage (1) oder händisch durch einen Handschuheingriff der Verarbeitungsanlage (1) und/oder mit einem bewegbaren Roboterarm und/oder mit einem magnetischen Fördersystem bestimmungsgemäß positioniert wird.

30. Verfahren nach dem vorhergehenden Anspruch, **dadurch gekennzeichnet, dass** die Ventilationsmaschine (4) nach der Einnahme der bestimmungsgemäßen Position für eine vorbestimmte Dauer ruht, bevor die Atmosphäre in der kontrollierten Umgebung (2) bewegt wird.

31. Verfahren nach einem der vorhergehenden Ansprüche, **dadurch gekennzeichnet, dass** mittels der Ventilationsmaschine (4) die kontrollierte Umgebung (2) gereinigt wird, insbesondere wobei mit der Ventilationsmaschine (4) eine turbulente Strömung erzeugt wird.

32. Verfahren nach einem der vorhergehenden Ansprüche, **dadurch gekennzeichnet, dass** eine Flüssigkeit auf Wasserbasis, insbesondere Wasser oder eine wässrige Lösung, in die kontrollierte Umgebung (2), insbesondere in die Ventilationsmaschine (4), eingebracht wird und die Flüssigkeit auf Wasserbasis mittels der Ventilationsmaschine (4) verteilt wird.

33. Verfahren nach einem der vorhergehenden Ansprüche, **dadurch gekennzeichnet, dass** mittels der Ventilationsmaschine (4) eine vorbestimmte Feuchtigkeit in der kontrollierten Umgebung (2) erzeugt wird.

34. Verfahren nach einem der vorhergehenden Ansprüche, **dadurch gekennzeichnet, dass** mittels der Ventilationsmaschine (4) die kontrollierte Umgebung (2) dekontaminiert wird, insbesondere wobei eine Dekontaminationssubstanz in die kontrollierte Umgebung (2) eingebracht und diese mittels der Ventilationsmaschine (4) verteilt wird.

35. Verfahren nach dem vorhergehenden Anspruch, **dadurch gekennzeichnet, dass** die Dekontaminationssubstanz in flüssiger Form eingebracht und vor dem Verteilen vaporisiert, insbesondere ionisiert wird, insbesondere wobei das Vaporisieren durch eine Ultraschallbehandlung und/oder ein Erhitzen der flüssigen Dekontaminationssubstanz herbeigeführt wird.

36. Verfahren nach einem der vorhergehenden Ansprüche, **dadurch gekennzeichnet, dass** die Drehrichtung (a, b) des Rotors (6) insbesondere wiederkehrend gewechselt wird.

37. Verfahren nach einem der Ansprüche 34 bis 36, **dadurch gekennzeichnet, dass** nach dem Verteilen der Dekontaminationssubstanz eine reine Atmosphäre mit der Ventilationsmaschine (4) verteilt wird, insbesondere um die kontrollierte Umgebung (2) zu spülen.

## Geänderte Patentansprüche

### Geänderte Patentansprüche gemäss Regel 137(2) EPÜ.

1. Verarbeitungsanlage (1) mit einer kontrollierten Umgebung (2) und einer die kontrollierte Umgebung (2) umgebenden Einhausung (3), wobei die Verarbeitungsanlage (1) mindestens eine mit der kontrollierten Umgebung (2) fluidal verbundene Ventilationsmaschine (4) mit einem Stator (5) und einem magnetisch berührungslos gelagerten Rotor (6) aufweist **dadurch gekennzeichnet, dass** die Ventilationsmaschine (4) derart ausgebildet und angeordnet ist, dass eine turbulente Strömung in der kontrollierten Umgebung (2) erzeugbar ist und/oder dass die Ventilationsmaschine (4) in der Einhausung (3), die die kontrollierte Umgebung umgibt, insbesondere in einer Hauptkammer (31), in welcher eine Verarbeitung einer Substanz, eines Materials, einer Probe oder ähnlichem erfolgt, angeordnet ist.

2. Verarbeitungsanlage (1) dem vorhergehenden Anspruch, **dadurch gekennzeichnet, dass** die Ventilationsmaschine (4) ein Bestandteil einer Dekontaminationseinrichtung ist.

3. Verarbeitungsanlage (1) nach einem der vorhergehenden Ansprüche, **dadurch gekennzeichnet, dass** die Ventilationsmaschine (4) einen Auslass (18), insbesondere eine Einstoffdüse oder eine Zweistoffdüse, aufweist, aus welchem eine Dekontaminationssubstanz, insbesondere Wasserstoffperoxid, in eine mit dem Stator (5) und/oder dem Rotor (6) erzeugte Strömung ausführbar ist.

4. Verarbeitungsanlage (1) nach einem der vorhergehenden Ansprüche, **dadurch gekennzeichnet, dass** der Stator (5) und/oder der Rotor (6) eine geschlossene Oberfläche aufweist/aufweisen.

5. Verarbeitungsanlage (1) nach einem der vorhergehenden Ansprüche, **dadurch gekennzeichnet, dass** die Verarbeitungsanlage eine Vibrationseinrichtung (19) zum Vaporisieren einer Dekontaminationssubstanz, insbesondere eine Ultraschalleinrichtung, aufweist.

6. Verarbeitungsanlage (1) nach einem der vorhergehenden Ansprüche, **dadurch gekennzeichnet, dass** die kontrollierte Umgebung (2) von der Einhausung (3) druckdicht umschließbar ist.

7. Verarbeitungsanlage (1) nach einem der vorhergehenden Ansprüche, **dadurch gekennzeichnet, dass** die kontrollierte Umgebung (2) eine Reinheit gemäß der Reinraumklasse A aufweist.

8. Verarbeitungsanlage (1) nach einem der vorhergehenden Ansprüche, **dadurch gekennzeichnet, dass** die Ventilationsmaschine (4) ein Bestandteil einer Reinigungseinrichtung ist.

9. Verarbeitungsanlage (1) nach einem der vorhergehenden Ansprüche, **dadurch gekennzeichnet, dass** Leitflächen zur Führung einer erzeugten Strömung ausgebildet sind.

10. Verarbeitungsanlage (1) nach einem der vorhergehenden Ansprüche, **dadurch gekennzeichnet, dass** die Verarbeitungsanlage eine Filtervorrichtung (30) zum Filtern von Partikeln aufweist.

11. Verarbeitungsanlage (1) nach dem vorhergehenden Anspruch, **dadurch gekennzeichnet, dass** der Stator (5) und/oder der Rotor (6) derart ausgebildet ist/sind, dass eine Strömung zumindest anteilig, insbesondere vollständig, radial von der Ventilationsmaschine (4) abgeblasen wird.

12. Verarbeitungsanlage (1) nach einem der vorhergehenden Ansprüche, **dadurch gekennzeichnet, dass** der Stator (5) und/oder der Rotor (6) derart ausgebildet ist/sind, dass eine Strömung zumindest anteilig, insbesondere vollständig, axial von der Ventilationsmaschine (4) abgeblasen wird.

13. Verarbeitungsanlage (1) nach einem der vorhergehenden Ansprüche, **dadurch gekennzeichnet, dass** der Rotor (6) derart ausgebildet ist, dass mit einer Drehung in einer ersten Drehrichtung (a) eine zumindest anteilig radial von der Ventilationsmaschine (4) abgeblasene Strömung erzeugbar ist und dass mit einer Drehung in einer zweiten Drehrichtung (b) eine zumindest anteilig axial von der Ventilationsmaschine (4) abgeblasene Strömung erzeugbar ist.

14. Verarbeitungsanlage (1) nach einem der vorgehenden Ansprüche, **dadurch gekennzeichnet, dass** der Stator (5) und der Rotor (6) derart ausgebildet sind, dass diese beiden Bauteile in einer einzigen Orientierung zueinander aneinander anordbar sind.

15. Verarbeitungsanlage (1) nach einem der vorhergehenden Ansprüche, **dadurch gekennzeichnet, dass** die Stator-und-Rotor-Kombination eine geometrische Codierung hat, insbesondere durch einen kegelstumpfförmig ausgebildeten Abschnitt (8, 13) und/oder einen gestuften Absatz (22) und eine dazu komplementäre Gegenform (14, 23, 24), insbesondere wobei der Rotor (6) in einem Mittelteil die komplementäre Gegenform (14, 23, 24) aufweist.

16. Verarbeitungsanlage (1) nach einem der vorhergehenden Ansprüche, **dadurch gekennzeichnet, dass** die Verarbeitungsanlage, insbesondere die Ventilationsmaschine (4), eine Beheizungseinrichtung (20) aufweist.

17. Verarbeitungsanlage (1) nach einem der vorhergehenden Ansprüche, **dadurch gekennzeichnet, dass** der Stator (5) als ein in die kontrollierte Umgebung (2) hineinragender Bestandteil der Einhausung (3) ausgebildet ist.

18. Verarbeitungsanlage (1) nach einem der vorhergehenden Ansprüche, **dadurch gekennzeichnet, dass** die Ventilationsmaschine (4) ein Gehäuse (26) aufweist.

19. Verarbeitungsanlage (1) nach einem der vorhergehenden Ansprüche, **dadurch gekennzeichnet, dass** die Einhausung (3) eine Hauptkammer (31) und mindestens eine fluidal abtrennbare Nebenkammer (32) aufweist, insbesondere wobei die Nebenkammer (32) eine Zuluftleitung, eine Abluftleitung und/oder eine Schleusenkammer ist, wobei die Ventilationsmaschine (4) in der Nebenkammer (32) angeordnet ist.

20. Verarbeitungsanlage (1) nach einem der vorhergehenden Ansprüche, **dadurch gekennzeichnet, dass** die Ventilationsmaschine (4) ortsveränderlich angeordnet ist.

21. Verarbeitungsanlage (1) nach einem der vorhergehenden Ansprüche, **dadurch gekennzeichnet, dass** die Ventilationsmaschine (4) auf einem auf einem magnetischen Fördersystem (35), insbesondere einem Magnetmover eines magnetischen Fördersystems (35), angeordnet ist.

22. Verarbeitungsanlage (1) nach einem der vorhergehenden Ansprüche, **dadurch gekennzeichnet, dass** die Ventilationsmaschine (4) an einem bewegbaren Roboterarm (38) angeordnet ist.

23. Verarbeitungsanlage (1) nach einem der vorhergehenden Ansprüche, **dadurch gekennzeichnet, dass** die Verarbeitungsanlage, insbesondere die Ventilationsmaschine (4), einen Energiespeicher (16) zur Versorgung der Ventilationsmaschine (4) mit elektrischer Energie aufweist.

24. Verarbeitungsanlage (1) nach einem der vorhergehenden Ansprüche, **dadurch gekennzeichnet, dass** der Stator (5), der Rotor (6) und/oder der Energiespeicher (16) eingekapselt ausgebildet ist/sind.

25. Verarbeitungsanlage (1) nach einem der vorhergehenden Ansprüche, **dadurch gekennzeichnet, dass** mindestens ein Bereich der Verarbeitungsanlage (1), insbesondere mindestens ein Bereich der Ventilationsmaschine (4), eine katalytische Beschichtung (21) aufweist.

26. Verarbeitungsanlage (1) nach einem der vorhergehenden Ansprüche, **dadurch gekennzeichnet, dass** die katalytische Beschichtung (21) ein Manganoxid aufweist oder ist.

27. Verfahren zum Behandeln einer kontrollierten Umgebung (2) einer Verarbeitungsanlage (1) nach einem der vorhergehenden Ansprüche, **dadurch gekennzeichnet, dass** mittels der Ventilationsmaschine (4) eine Atmosphäre in der kontrollierten Umgebung (2) bewegt wird.

28. Verfahren nach dem vorhergehenden Anspruch, **dadurch gekennzeichnet, dass** die Ventilationsmaschine (4) oder zumindest der Rotor (6) der Ventilationsmaschine (4) händisch durch eine Öffnung in einer Einhausung (3) der Verarbeitungsanlage (1) oder händisch durch einen Handschuheingriff der Verarbeitungsanlage (1) und/oder mit einem bewegbaren Roboterarm und/oder mit einem magnetischen Fördersystem bestimmungsgemäß positioniert wird.

29. Verfahren nach dem vorhergehenden Anspruch, **dadurch gekennzeichnet, dass** die Ventilationsmaschine (4) nach der Einnahme der bestimmungsgemäßen Position für eine vorbestimmte Dauer ruht, bevor die Atmosphäre in der kontrollierten Umgebung (2) bewegt wird.

30. Verfahren nach einem der vorhergehenden Ansprüche, **dadurch gekennzeichnet, dass** mittels der Ventilationsmaschine (4) die kontrollierte Umgebung (2) gereinigt wird, insbesondere wobei mit der Ventilationsmaschine (4) eine turbulente Strömung erzeugt wird.

31. Verfahren nach einem der vorhergehenden Ansprüche, **dadurch gekennzeichnet, dass** eine Flüssigkeit auf Wasserbasis, insbesondere Wasser oder eine wässrige Lösung, in die kontrollierte Umgebung (2), insbesondere in die Ventilationsmaschine (4), eingebracht wird und die Flüssigkeit auf Wasserbasis mittels der Ventilationsmaschine (4) verteilt wird.

32. Verfahren nach einem der vorhergehenden Ansprüche, **dadurch gekennzeichnet, dass** mittels der Ventilationsmaschine (4) eine vorbestimmte Feuchtigkeit in der kontrollierten Umgebung (2) erzeugt wird.

33. Verfahren nach einem der vorhergehenden Ansprüche, **dadurch gekennzeichnet, dass** mittels der Ventilationsmaschine (4) die kontrollierte Umgebung (2) dekontaminiert wird, insbesondere wobei eine Dekontaminationssubstanz in die kontrollierte Umgebung (2) eingebracht und diese mittels der Ventilationsmaschine (4) verteilt wird.

34. Verfahren nach dem vorhergehenden Anspruch, **dadurch gekennzeichnet, dass** die Dekontaminationssubstanz in flüssiger Form eingebracht und vor dem Verteilen vaporisiert, insbesondere ionisiert wird, insbesondere wobei das Vaporisieren durch eine Ultraschallbehandlung und/oder ein Erhitzen der flüssigen Dekontaminationssubstanz herbeigeführt wird.

35. Verfahren nach einem der vorhergehenden Ansprüche, **dadurch gekennzeichnet, dass** die Drehrichtung (a, b) des Rotors (6) insbesondere wiederkehrend gewechselt wird.

36. Verfahren nach einem der Ansprüche 33 bis 35, **dadurch gekennzeichnet, dass** nach dem Verteilen der Dekontaminationssubstanz eine reine Atmosphäre mit der Ventilationsmaschine (4) verteilt wird, insbesondere um die kontrollierte Umgebung (2) zu spülen.
